# EUROPEAN PATENT APPLICATION

(11) **EP 4 454 664 A2**
(43) Date of publication of application: **30.10.2024**
(21) Application number: 24195075.7
(22) Date of filing: 02.11.2017
(51) Int. Cl.: A61K 47/26

(54) **DISSOLVABLE FILMS AND METHODS OF THEIR USE**

(30) Priority: 02.11.2016 US 201662416394 P
(62) Divisional of application: 17867392.7
(71) Applicant: Board of Regents, The University of Texas System, Austin, TX 78701 (US)
(72) Inventor: CROYLE, Maria A., Austin, TX 78704 (US); SCHAFER, Stephen Clay, Austin, TX 78704 (US)
(74) Representative: Vossius & Partner Patentanwälte Rechtsanwälte mbB

(57) **Abstract**

The present invention relates to a composition comprising an amorphous, substantially solid film comprising a biologically active polypeptide, a sugar, and an amphipathic surfactant, wherein the biologically active polypeptide is selected from factors acting on the immune system, an anti-infective agent, an antibody, an enzyme, a ligand, and a receptor, and wherein the amphipathic surfactant comprises poly(maleic anhydride-alt-1 octadecene) substituted with 3-(dimethylamino) propylamine.

## Description

This application claims the benefit of United States Provisional Patent Application No 62/416,394, filed November 2, 2016, the entirety of which is incorporated herein by reference.

The invention was made with government support under Grant No. U01 AI078045 awarded by the National Institutes of Health. The government has certain rights in the invention.

### BACKGROUND OF THE INVENTION

### 1. Field of the Invention

The present invention relates generally to the fields of biotechnology and pharmaceutical chemistry. More particularly, it concerns compositions and methods for the delivery of biologically active polypeptides.

### 2. Description of Related Art

Pharmaceutically active polypeptides are known to be useful in the medical field in a variety of therapies. Advances in biotechnology have enabled large-scale production of natural and recombinant polypeptides for manufacturing products in the pharmaceutical industry. The use of some polypeptides has, however, been limited by their limited stability at ambient temperatures. Accordingly, most polypeptide-based therapies must be refrigerated to maintain their stability. To date this has been a severe limitation the use of recombinant polypeptides, which would otherwise represent a potentially huge new pool of disease therapy candidates. In some embodiments, the instant patent application provides, for the first time new methodologies for stabilizing polypeptides molecules that addresses the problems regarding their limited stability.

### SUMMARY OF THE INVENTION

In a first embodiment, the present disclosure provides a composition comprising an amorphous, substantially solid film having an average thickness of 0.005 to 5mm, which is soluble in an aqueous solution, said film comprising a biologically active polypeptide, a sugar and an amphipathic surfactant. In some aspects, a film of the embodiments has an average thickness of 0.005, 0.01, 0.1, 0.2, 0.3, 0.4, or 0.5 to about 1, 1.5 or 2 mm. For example, the film may comprise an average thickness of 0.1 to 2 mm or 0.5 to 2 mm.

In some aspects, the sugar for use according to the embodiments is selected from group consisting of: glucose, dextrose, fructose, lactose, maltose, xylose, sucrose, corn sugar syrup, sorbitol, hexitol, maltilol, xylitol, mannitol, melezitose, raffinose, and/or a combination thereof. In particular aspects, the amphipathic surfactant comprises poly(maleic anhydride-alt-1 octadecene). In one specific aspect, the poly(maleic anhydride-alt-1 octadecene) is substituted with 3-(dimethylamino) propylamine. In some aspects, the composition comprises an amphipathic surfactant (such as poly(maleic anhydride-alt-1 octadecene)) at a concentration of about 0.1 to 50 mg/ml, 1 to 40 mg/ml, 1 to 30 mg/ml, 1 to 20 mg/ml, or 5 to 15 mg/ml (e.g., about 10 mg/ml). In some aspects, the composition comprises about 0.1% to 10%, 0.5% to 10%, 0.5% to 5%, 1% to 10%, or 1% to 5% of an amphipathic surfactant (e.g., poly(maleic anhydride-alt-1 octadecene)). In further aspects, the composition also comprises a pH buffering agent (e.g., phosphate buffered saline). In certain aspects, the carrier has a pH of between 5.0 and 8.0, between 5.5 and 8.0, between 6.0 and 8.0, between 6.0 and 7.5 or between 6.1 and 7.4. In certain aspects, the composition further comprises a buffer. In some aspects, the composition further comprises a water-soluble polymer. In additional aspects, the composition further comprises a salt. Additional components for use in films of the embodiments are provided in U.S. Patent Publication No. 20160296616, which is incorporated herein by reference.

In further aspects, a composition of the embodiments comprises Hydroxypropyl Methylcellulose (HPMC). For example, in some aspects, a composition comprises (or is dried down from a composition that comprises) about 0.01% to 10%, 0.1% to 7%, 0.5% to 5%, 1% to 5%, or 1% to 3% HPMC (e.g., in some aspects a composition is dried down from a solution comprising about 1.5 % HPMC). In yet further aspects, a composition of the embodiments comprises tragacanth gum. For example, in some aspects, a composition comprises (or is dried down from a composition that comprises) about 0.01% to 5%, 0.01% to 2%, 0.05% to 1%, 0.05% to 0.5%, or 0.1% to 1.5% tragacanth gum (e.g., in some aspects a composition is dried down from a solution comprising about 0.2 % tragacanth gum). In still further aspects, a composition of the embodiments comprises sorbitol. For example, in some aspects, a composition comprises (or is dried down from a composition that comprises) about 0.01% to 10%, 0.1% to 7%, 0.5% to 5%, 1% to 5%, or 1% to 3% sorbitol *(e.g.,* in some aspects a composition is dried down from a solution comprising about 2 % sorbitol).

In particular aspects, the biologically active polypeptide does not comprise an antigen, such as a cancer cell antigen or an infectious disease antigen, such as a viral, bacterial or parasite antigen. In some aspects, the biologically active polypeptide does not comprise a virus or a virus vector. In some aspects, the biologically active polypeptide is substantially non-immunogenic when administered to a human. In particular aspects, the biologically active polypeptide is a human polypeptide or a humanized antibody. In certain aspects, the biologically active polypeptide is PEGylated. In some aspects, the biologically active polypeptide is an antibody, such as a monoclonal antibody. In particular aspects, the monoclonal antibody is a humanized antibody. In other aspects, the biologically active polypeptide is an enzyme, a ligand or a receptor. In specific aspects, the biologically active polypeptide is an enzyme or a cytokine. In some aspects, the biologically active polypeptide is the extra cellular domain of a receptor.

In yet further aspects, a composition of the embodiments is defined as able to retain at least about 70%, 75%, 80%, 85% 90%, 95%, 96%, 97% or 98% *(e.g.,* 70-95%) of the starting activity (e.g., signaling activity or enzymatic activity) of the biologically active polypeptide after storage at room temperature for 1 week, 2, weeks, 3 weeks, 1 month, 2 months, 4 months, 6 months, 8 months or 1 year or more.

In a further embodiment, the composition (e.g., comprising an amorphous, substantially solid film having an average thickness of 0.005 to 5mm) is produced by a method comprising providing an aqueous solution comprising the biologically active polypeptide, the sugar and the amphipathic surfactant, and drying the solution sufficiently to form an amorphous, substantially solid film, which is soluble in an aqueous solution. In some aspects, the drying step is performed at an ambient temperature. In further aspects, the drying step is performed between about 20°C and 40°C or between 20°C and 30°C. In some aspects, prior to drying, the solution comprises about 0.1 to 50 mg/ml, 1 to 40 mg/ml, 1 to 30 mg/ml, 1 to 20 mg/ml, or 1 to 10 mg/ml of an amphipathic surfactant, such as poly(maleic anhydride-alt-1 octadecene).

Another embodiment provides a composition comprising an amorphous substantially solid film comprising a biologically active polypeptide, a sugar and an amphipathic surfactant, said composition produced by a method comprising providing an aqueous solution comprising the biologically active polypeptide, the sugar and the amphipathic surfactant, and drying the solution sufficiently to form an amorphous, substantially solid film, which is soluble in an aqueous solution. In certain aspects, the drying step is performed at an ambient temperature. In particular aspects, the drying step is performed at 0.1 to 3.0 atm, 0.5 to 2.0 atm or 0.8 to 1.5 atm of pressure.

In yet another embodiment, there is provided a method of providing biologically active polypeptide to a subject comprising administering an effective amount of a composition of the embodiments. For example, the composition for administration can comprise an amorphous, film as detailed above or an aqueous composition comprising the film components, such as a biologically active polypeptide, a sugar and an amphipathic surfactant (e.g., a film reconstituted in a liquid). In some aspects, the method further comprises reconstituting the composition in an aqueous solution prior to administration. In certain aspects, the aqueous solution is administered *via* injection *(e.g.,* by intravenous, intramuscular, intradermal or subcutaneous injection). In particular aspects, the aqueous solution is sterile. In one specific aspects, the aqueous solution is a pH buffered solution, such as solution having pH of between about 6.0 and 8.0. In certain aspects, the method comprises administering the composition orally, sublingually or intranasally.

In general, film composition of the embodiments may be formulated so as to dissolve in a relatively short period of time. For example, the film may dissolve in water (or a buffer aqueous solution) in from about 1 to 10 seconds, 1-10 minutes or one hour or less. In some aspects, dissolving a film comprises mechanically oscillating the film in the solution (e.g., by shaking or vortexing the solution and film). In some instances, the films may be fully dissolvable in water in 1 - 24 hours. Dried films, in some embodiments, will have a final moisture content of 0.1-10% *(e.g.,* 0.1-5%, 0.1 -3% or 0.1 to 1%) water as determined by standard techniques. In some embodiments moisture content of a film maybe as high as 20 - 40% depending upon the types of excipients and the antibody/enzyme included in the film matrix. The final pH of the dried film, preferably is within the range of 4.0-8.0, 6.0-8.0, 7.0-8.0 or 4-7.4.

Certain aspects of the embodiments concern dried-down compositions. As used herein a dried-down composition refers to, *e.g.,* a film such as those described herein, preferably having less than 5%, or more preferably less than 1% water content. Accordingly, a skilled worker will recognize that other components of a solution that is dried-down will be concentrated in the resulting dried-down films.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore below 0.01%. Most preferred is a composition in which no amount of the specified component can be detected with standard analytical methods.

As used herein in the specification and claims, "a" or "an" may mean one or more. As used herein in the specification and claims, when used in conjunction with the word "comprising", the words "a" or "an" may mean one or more than one. As used herein, in the specification and claim, "another" or "a further" may mean at least a second or more.

As used herein in the specification and claims, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating certain embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

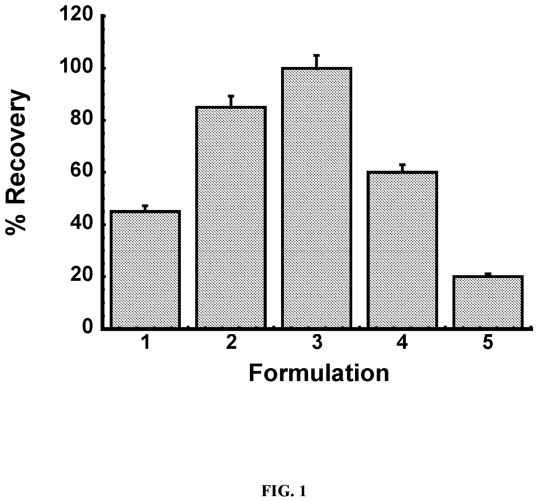

FIG. 1 - Various Formulations Preserve Antibody and Enzymatic Activity After Drying and Storage in a Thin Film Matrix. A horseradish peroxidase conjugated goat anti-mouse IgG secondary antibody was mixed with a variety of formulations and dried in a thin film matrix at room temperature. Forty-eight hours after drying was complete, the film was reconstituted with buffer commonly utilized for Western Blots. The reconstituted solutions were incubated with nylon membranes containing murine liver lysate previously incubated with an IgG anti-mouse beta actin primary antibody. Membranes were then incubated with a horseradish peroxidase substrate and immune complexes detected by chemiluminescence. The intensity of protein bands generated by secondary antibody dried in the film matrix and subsequently reconstituted were compared to those generated by secondary antibody stored in the manufacturer's liquid formulation at 4°C , Data is reported as percent recovery of activity.

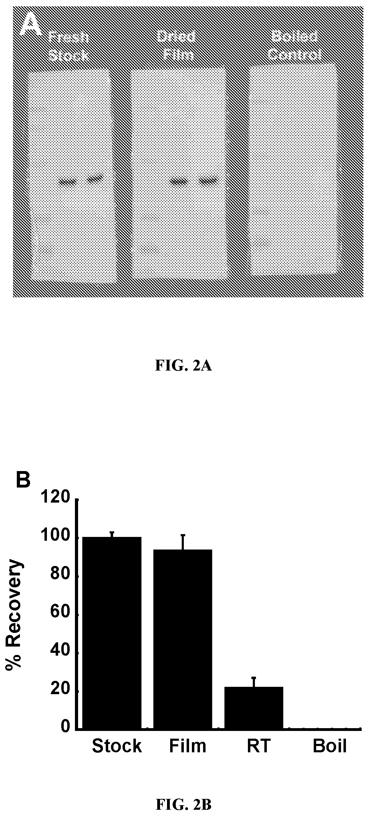

FIGS. 2A-2B - Film Technology Preserves Ability of Antibodies to Bind to Target and Activity of A Peroxidase Enzyme After Storage at Room Temperature (21 °C). A) Representative Western Blot performed with a horseradish peroxidase conjugated goat anti-mouse IgG secondary antibody stabilized in film matrix consisting of 1.5% w/w HPMC/2% w/v sorbitol/0.2% w/v tragacanth gum in 100 mM phosphate buffered saline pH 7.4 and stored at 20°C for 3 months prior to reconstitution. B) Graph depicting similar band densities generated for antibody dried in film matrix and stored at 20 °C for 90 days and standard stock stored at 4°C. Boil = Negative Control. Antibody heated to 65 °C for 30 minutes prior to use in Western Blot. RT= Band Density for antibody stock from manufacturer stored at room temperature for 24 hours.

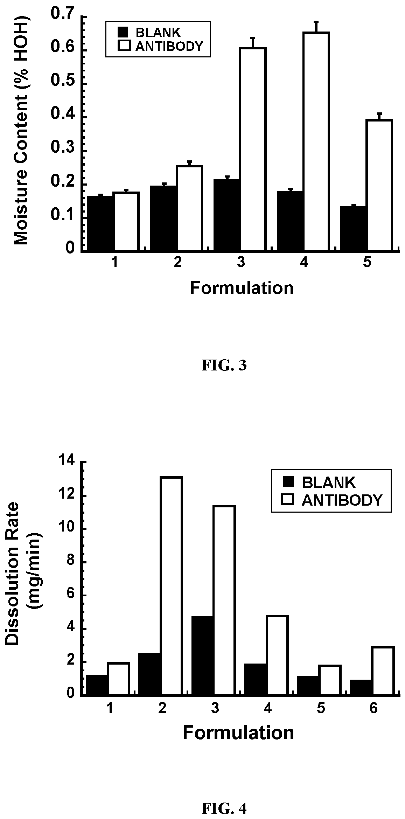

FIG. 3 - Antibody Influences the Moisture Retained in Dried Film Formulations. Moisture content for films of uniform weight containing 10 micrograms of a monoclonal antibody (ANTIBODY)/ /mm³ film and blank controls was assessed by Karl Fischer titration according to USP standards. While formulations alone (blank) did not significantly vary in moisture content after drying was complete, they did influence the ability of the embedded antibody to retain moisture in the dried state to varying degrees. Formulations evaluated were as follows: (1) 0.5% w/w HPMC, (2) 0.5% w/w HPMC/2% w/v sorbitol, (3) 0.5% w/w HPMC/2% v/v glycerol, (4) 1.5% w/w HPMC/2% v/v glycerol, (5) 3% w/w HPMC/2% w/v sorbitol. All films contained 0.2% w/v tragacanth gum and were buffered in 10 mM Tromethamine USP, pH 8.0. The drying process for all films was complete within 5 hours.
FIG. 4 - Antibody Significantly Impacts Dissolution Rate of Films in Simulated Saliva. Films of uniform weight containing 50 micrograms of a monoclonal antibody (ANTIBODY) per mm³ of film and blank controls were placed in simulated human salivary fluid at 37 °C under gentle stirring. Dissolution rate was calculated by dividing the starting weight of the film by the time at which the film could no longer be visibly detected in the solvent. Formulations alone (blank) provided a variety of dissolution rates after drying was complete. These differences were enhanced in the presence of antibody. Formulations evaluated were as follows: (1) 0.5% w/w HPMC/2% w/v sorbitol, (2) 0.5% w/w HPMC/2% w/v glycerol, (3) 1.5% w/w HPMC/2% w/v sorbitol, (4) 1.5% w/w HPMC/2% v/v glycerol, (5) 3% w/w HPMC/2% w/v sorbitol, (6) 3% HPMC/2% v/v glycerol. Each film contained 0.2% w/v tragacanth gum and was buffered in 100 mM phosphate buffered saline, pH 7.4. Drying for all films was complete in 4 hours. Simulated human saliva fluid consisted of: KCl 0.15 g/L, NaCl 0.12 g/L, Sodium Bicarbonate 2.1 g/L, alpha-amylase 2.0 g/L and gastric mucin 1.0 g/L as described in Davis et al. 1971.

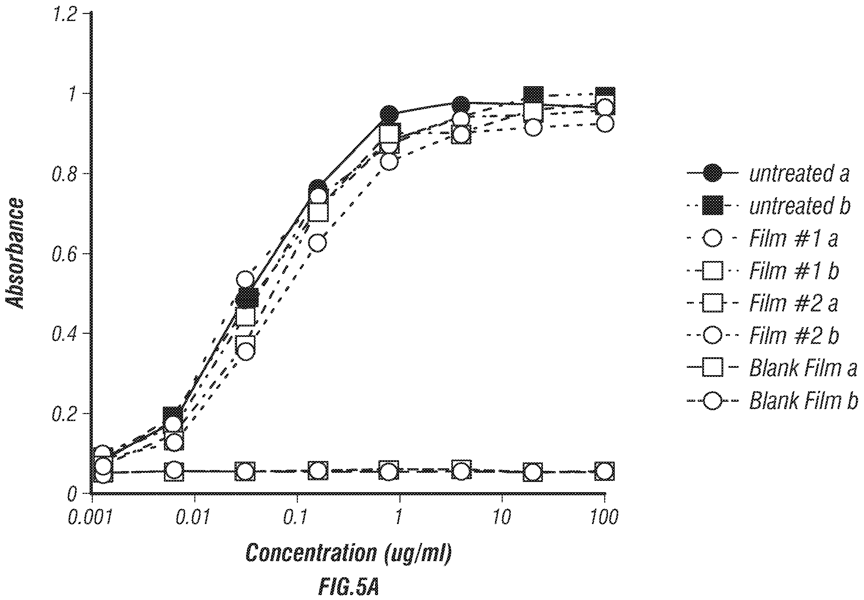

FIGS. 5A-5B - Film Technology Preserves Binding Affinity of Humanized IgG1 Anti-Pertussis Toxin Antibody (hu11E6) to Human Pertussis Toxin and Prevents Aggregation in Solution. Hu11E6 was mixed with a formulation containing 1.5% HPMC, 0.2% tragacanth gum, 2% sorbitol and PMAL C16 (1%), pH 8.1 and dried in a thin film matrix at 20°C for 14 hours. Once drying was complete, films were reconstituted with sterile water for injection. A) Indirect ELISA comparing binding affinities of hu11E6 prior to (untreated) and upon reconstitution of dried film (Film 1, 2). B) Chromatograph of purified hu11E6 (untreated) and hu11E6 after reconstitution from the thin film matrix. A single peak for each preparation indicates the absence of aggregates in the final product. Size Exclusion Chromatography was performed using an ActaPure S200 system with a 100 µl loop.

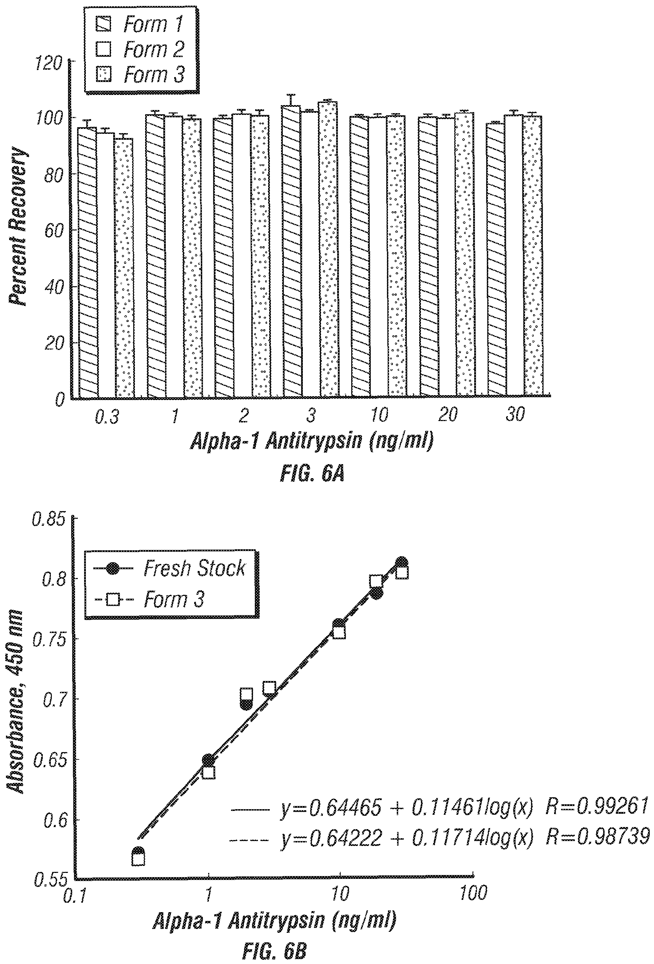

FIGS. 6A-6B - Film Technology Preserve Binding Affinity of a Primary Antibody in an Alpha-1 Antitrypsin (A1AT) ELISA Assay. A monoclonal mouse anti-human alpha-1 antitrypsin antibody (178260 Millipore) was mixed with three different formulations. Films were dried under ambient conditions overnight and reconstituted the following day. Reconstituted solutions were utilized in an A1AT ELISA assay (see, Le *et al.,* 2005, incorporated herein by reference). A) Recovery of binding affinity of 178260 for alpha-1 antitrypsin upon reconstitution of thin film matrix. Percent recovery is the relative absorbance reading generated by an assay utilizing 178260 from reconstituted films with that of fresh 178260 as supplied by the manufacturer for each given concentration of A1AT standard. Data reflect the results from 3 separate experiments including triplicate samples for each formulation. Formulation 1: 3% HPMC, 0.2% tragacanth gum, 10% trehalose. Formulation 2: 1.5% HPMC, 0.2% tragacanth gum, 5% sucrose, 1% PEG 3000. Formulation 3: 1.5% HPMC, 0.2% tragacanth gum, 2% sorbitol, 1% PMAL C16. All formulations utilized in films to generate data for this figure were prepared in 10mM Tris, pH 8.1 B) Representative standard curve generated with fresh 178260 (Fresh stock) and antibody stabilized in a thin film matrix (Form 3).

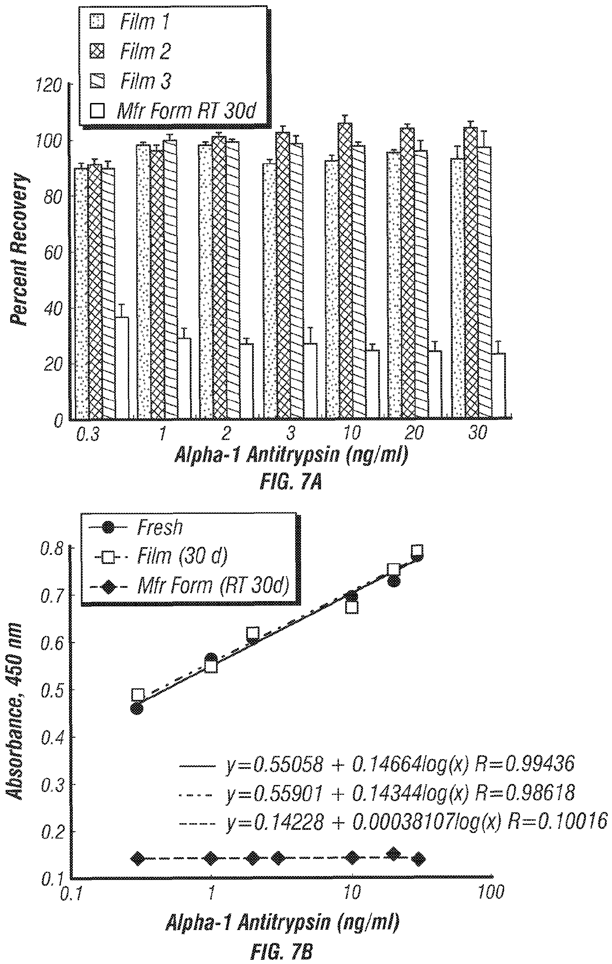

FIGS. 7A-7B - Binding Affinity of a Primary Antibody (178260) Stabilized in a Thin Film Matrix and Stored at Room Temperature (RT) for 30 days is Superior to that of the Manufacturer's Product Stored Under the Same Conditions. A monoclonal mouse anti-human alpha-1 antitrypsin antibody (178260 Millipore) was mixed with a formulation containing 1.5% HPMC, 0.2% tragacanth gum, 2% sorbitol and 10mg/ml PMAL C16. Films were dried under ambient conditions overnight and stored at 20°C for 30 days. Reconstituted solutions were utilized in an A1AT ELISA assay (Le *et al.,* 2005). A) Recovery of binding affinity of 178260 for alpha-1 antitrypsin upon reconstitution of thin film matrix. Percent recovery is the relative absorbance reading generated by an assay utilizing 178260 from reconstituted films with that of fresh 178260 as supplied by the manufacturer for each given concentration of A1AT standard. Results were also compared with manufacturer's stock stored at RT (instead of 4 °C as recommended). Data reflect the results from 3 separate experiments. B) Representative standard curve generated with fresh 178260, antibody stored at RT in film for 30 days and stock stored at RT.

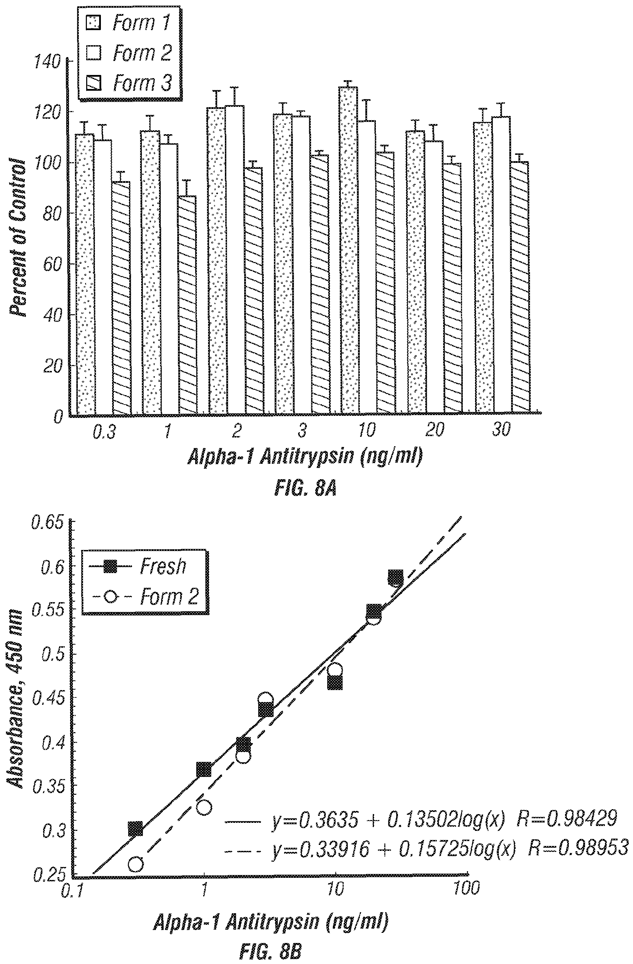

FIGS. 8A-8B - Film Technologies Do Not Impact Performance of a Horseradish Peroxidase Conjugated Secondary Antibody in a Novel Alpha-1 Antitrypsin (A1AT) ELISA Assay. A polyclonal donkey horseradish peroxidase conjugated anti-mouse IgG antibody (AP192P EMD Millipore) was mixed with three different formulations developed to stabilize biologicals in a thin film matrix. Formulation 1: 3% HPMC, 0.2% tragacanth gum , 20% sucrose in 100 mM phosphate buffered saline (pH 7.4). Formulation 2: 1.5% HPMC, 0.2% tragacanth gum, 5% sucrose , 2% PEG 5000 in 10 mM Tris buffer (pH 8.1). Formulation 3: 1.5% HPMC, 0.2% tragacanth gum , 10% melezitose in 50 mM citrate buffer (pH 5.5). These preparations were utilized in an A1AT ELISA to determine if excipients would interfere with the readout of the assay. A) Binding affinity of AP192P for primary antibody utilized in the ELISA assay. Percent recovery is the relative absorbance reading generated by AP192P in various formulations with that of the same antibody in the formulation supplied by the manufacturer for each given concentration of A1AT standard. Data reflect the results from 3 separate experiments including triplicate samples for each formulation. B) Representative standard curve generated with fresh AP192P (Fresh stock) and antibody in a novel formulation (Form 2).

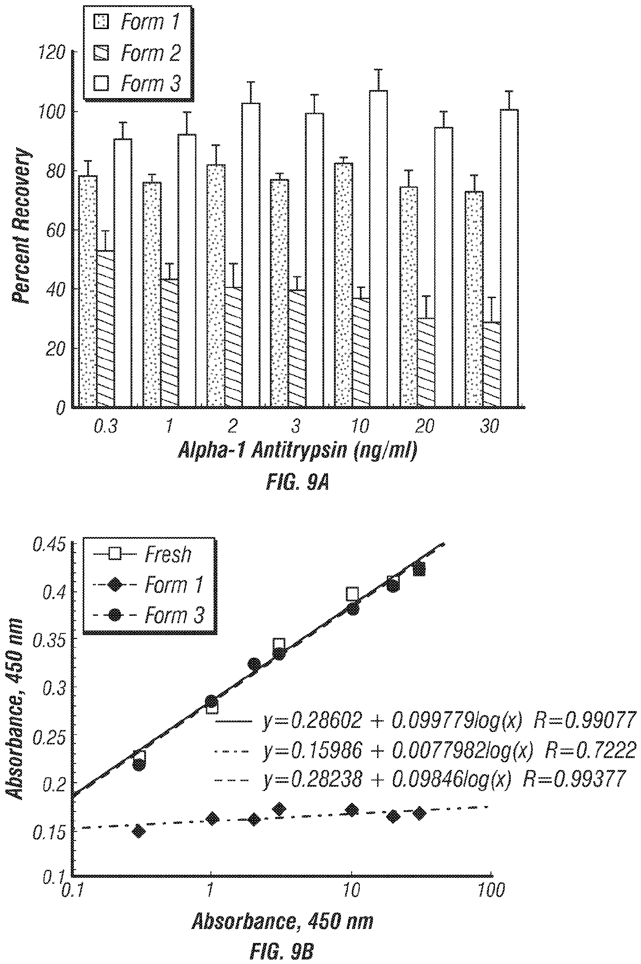

FIGS. 9A-9B - Film Technologies Preserve Binding Affinity of a Horseradish Peroxidase Conjugated Secondary Antibody in an Alpha-1 Antitrypsin (A1AT) ELISA Assay. AP192P was mixed with three different formulations. Formulation 1 3% HPMC, 0.2% tragacanth gum, 40 mg/ml mannitol, 10 mg/ml sucrose and 10 mg/ml PMAL C16 in 100 mM phosphate buffered saline (pH 7.4). Formulation 2: 1.5% HPMC, 0.2% tragacanth gum , 10% melezitose in 50 mM citrate buffer (pH 5.5). Formulation 3: 1.5% HPMC, 0.2% tragacanth gum, 2% sorbitol, 1% PMAL C16 in 10mM Tris buffer (pH 8.1). Films were dried under ambient conditions overnight and reconstituted the following day. Reconstituted solutions were utilized in an A1AT ELISA (Le *et al.,* 2005). A) Recovery of binding affinity of AP192P for a primary mouse anti-human alpha-1 antitrypsin antibody (178260) upon reconstitution of thin film matrix. Percent recovery is the relative absorbance reading generated by an assay utilizing AP192P from reconstituted films with that of fresh AP192P as supplied by the manufacturer for each given concentration of A1AT standard. Data reflect the results from 3 separate experiments including triplicate samples for each formulation. B) Representative standard curve generated with fresh AP192P (Fresh) and antibody stabilized in 2 different thin film matrices (Form 1 and 3).

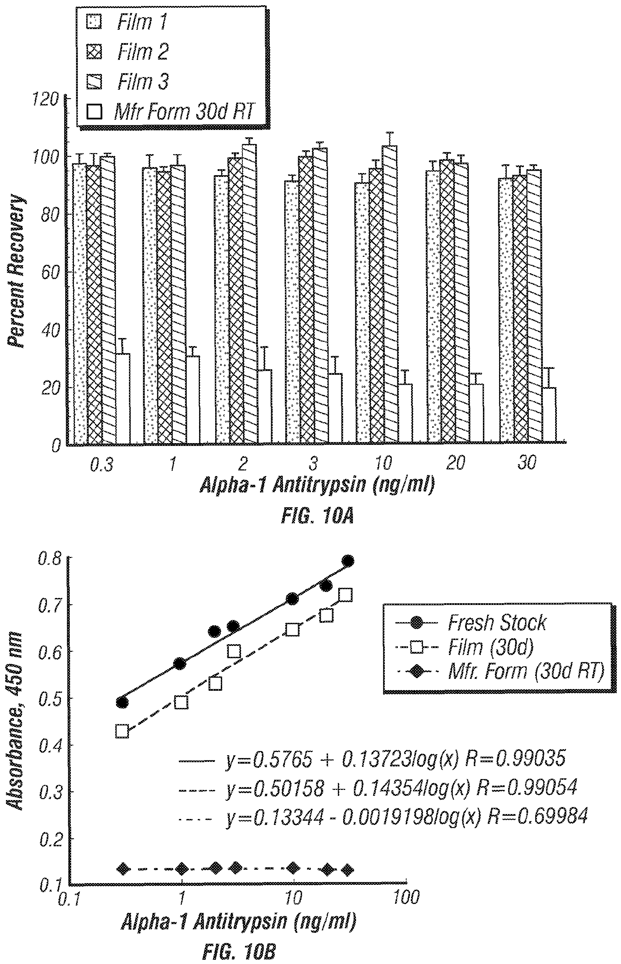

FIGS. 10A-10B - Binding Affinity of AP192P Stabilized in a Thin Film Matrix and Stored at Room Temperature (RT) for 30 days is Superior to that of the Manufacturer's Product Stored Under the Same Conditions AP192P was mixed with a formulation containing 1.5% HPMC, 0.2% tragacanth gum, 2% sorbitol and 10mg/ml PMAL C16 in 10mM Tris buffer (pH 7.4). Films were dried under ambient conditions overnight and stored at 20°C for 30 days. Reconstituted solutions were utilized in an A1AT ELISA. A) Recovery of binding affinity of AP192P for a primary mouse anti-human alpha-1 antitrypsin antibody reconstitution of thin film matrix. Percent recovery is the relative absorbance reading generated by an assay utilizing AP192P from reconstituted films with that of fresh AP192P as supplied by the manufacturer for each given concentration of A1AT standard. Results were also compared with manufacturer's stock stored at RT (instead of -20 °C as recommended). Data reflect the results from 3 separate experiments. B) Representative standard curve generated with fresh AP192P, antibody stored at RT in film for 30 days and stock stored at RT.

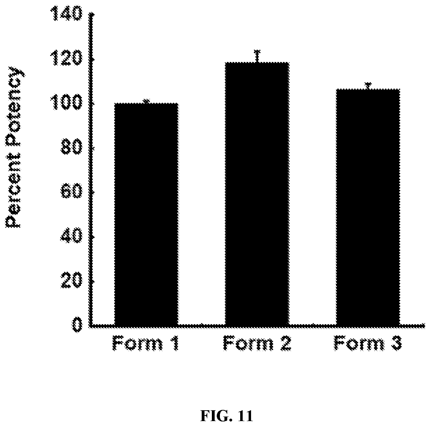

FIG. 11 - Streptokinase Activity is Preserved During the Film Forming Process. Streptokinase C isolated from the Streptococcus species (Sigma S0827) at a dose of 4 International Units (IU) was mixed with 3 different formulations and dried at room temperature for 16 hours. Once drying was complete, films were then reconstituted with a buffer consisting of 10 mM Tris (pH 7.4), 0.1M NaCl and human albumin (1 mg/ml). Streptokinase activity present in each reconstituted film was measured using a standard chromogenic assay (Sands *et al.,* 2004; and Mahboubi *et al.,* 2012, each incorporated herein by reference). Percent potency is the activity of streptokinase in the film with respect to freshly thawed enzyme measured in the same assay plate. Formulations included in this study were: Formulation 1 1.5% HPMC, 0.2% tragacanth gum, 5% sucrose, PMAL C16 (1%) in 0.1 M phosphate buffered saline (pH 7.4). Formulation 2: 3% HPMC, 0.2% tragacanth gum, 2% sorbitol, PMAL C16 (1%) in 0.1M citrate buffer (pH 5.5). Formulation 3: 1.5% HPMC, 0.2% tragacanth gum, 5% trehalose, PMAL C16 (1%) in 10 mM Tris buffer (pH 8.1). Data reflect the average streptokinase activity obtained from six individual films for each formulation. Error bars reflect the standard deviation of the data.

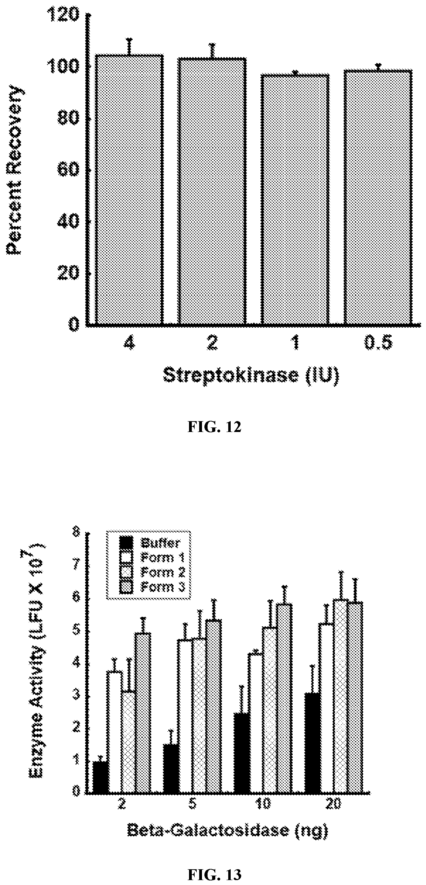

FIG. 12 - The Amount of Streptokinase Incorporated in a Film Does not Significantly Impact Stability in Thin Films. Several different amounts of Streptokinase C isolated from the Streptococcus species (Sigma S0827) were placed in a formulation consisting of 1.5% HPMC, 0.2% tragacanth gum, 2% sorbitol, PMAL C16 (1%) in 10 mM Tris buffer (pH 8.1). The activity of streptokinase in the preparation was determined prior to drying by a standard chromogenic assay (3, 4). Aliquots of the preparation were placed in unit dose molds and dried at 20°C for 17 hours. Once drying was complete, films were stored in sealed pouches at 25°C for 15 days. At that time, films were reconstituted with a buffer consisting of 10 mM Tris (pH 7.4), 0.1M NaCl and human albumin (1 mg/ml) and streptokinase activity present in each reconstituted film measured. Percent potency is the activity of streptokinase in the film after 15 days of storage with respect to activity measured in the formulation prior to drying. Data reflect the average streptokinase activity obtained from eight individual films for each amount of streptokinase indicated below. Error bars reflect the standard deviation of the data. It is also important to note that streptokinase activity could not be detected in manufacturer's samples (ie enzyme in phosphate buffer alone, pH 7.5) stored under the same conditions (data not shown).
FIG. 13 - Film Technologies Do Not Impact Enzymatic Activity of Beta-Galactosidase. Various amounts of β-Galactosidase from Escherichia coli (Grade VIII, 500 Units/mg protein) were mixed with 3 different formulations developed to stabilize biologicals in a thin film matrix. The enzymatic activity present in each formulation was assessed with a Galacto Light Plus Detection System (Applied Biosystems). Data reflect the results from 3 separate experiments including triplicate samples for each formulation. Performance of the enzyme was not significantly impacted by any of the formulations tested. Buffer: Recommended buffer for use for beta-galactosidase assays: 50mM Tris, pH 7.3. Formulation 1: 1.5% HPMC, 0.2% tragacanth gum, 2% sorbitol, 1% PMAL C16 in 100mM phosphate buffered saline (pH 7.4). Formulation 2: 1.5% HPMC, 0.2% tragacanth gum, 2% sorbitol, 1% PMAL C16 in 50mM citrate buffer (pH 5.5). Formulation 3: 1.5% HPMC, 0.2% tragacanth gum, 2% sorbitol, 1% PMAL C16 in 10 mM Tris buffered saline (pH 7.8).

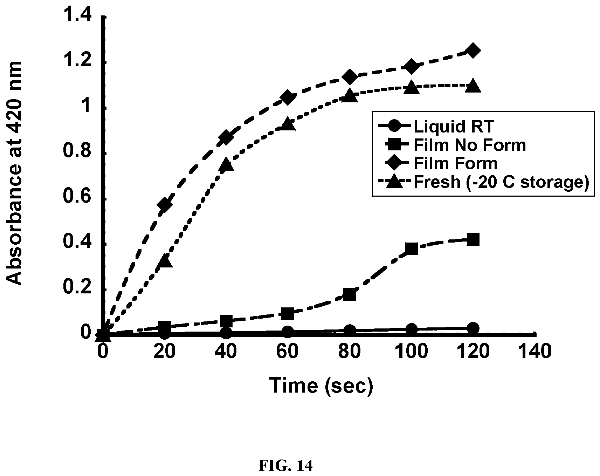

FIG. 14 - Film Technology Preserves Enzymatic Activity After Storage at 25°C for 10 days. Recombinant beta-galactosidase (MP Biomedicals, 10 mg, 300 units/mg) was incorporated into film matrix (3% HPMC/2% glycerol) in 10 mM phosphate buffered saline pH 7.0, dried under ambient conditions and stored at room temperature for 10 days. Films were then reconstituted and added to a solution consisting of 4 mg/ml *o*-nitrophenyl-β-D-galactoside (ONPG), 1mM MgCl₂ and 50 mM beta-mercaptoethanol (pH 7). Samples were placed in a plate reader and absorbance at 420 nm recorded every 20 seconds for 2 minutes. When the enzyme was stored in phosphate buffer (pH 7.4) alone at room temperature (LIQUID RT), very little enzymatic activity was detected at the 10 day time point. This is not surprising, since the manufacturer states that the enzyme will remain active for only 20 hours under these conditions. Beta-galactosidase dried in buffer and 1.5% HPMC (FILM no FORM) demonstrated more activity than the liquid formulation, however this was significantly lower than what was seen from antibody stored frozen as recommended by the manufacturer (FRESH -20 °C Storage). Reaction rates for enzyme in each of the described formulations are summarized in Table 1.

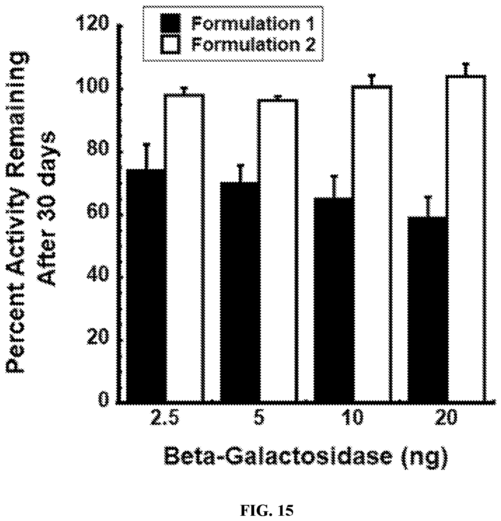

FIG. 15 - Optimized Formulations Preserve Enzymatic Activity of Beta-Galactosidase in a Thin Film Matrix for 30 Days at Room Temperature. Various amounts of β-Galactosidase from Escherichia coli (Grade VIII, 500 Units/mg protein) were mixed with 2 different formulations. Films were dried at ambient temperature for 16 hours and then stored at 20 °C prior to reconstitution. The enzymatic activity present in each reconstituted film was assessed with a Galacto Light Plus Detection System (Applied Biosystems). Data reflect the results from 3 separate experiments including triplicate samples for each formulation. Formulation 1: 3% HPMC, 0.2% tragacanth gum, 5% melezitose, 1% PEG 3000 in 100mM phosphate buffered saline (pH 7.4). Formulation 2: 1.5% HPMC, 0.2% tragacanth gum, 2% sorbitol, 1% PMAL C16 in 10mM Tris buffer (pH 8.1).

### DESCRIPTION OF ILLUSTRATIVE EMBODIMENTS

### I. The Present Embodiments

The present disclosure generally relates to biologically active polypeptide compositions stabilized in a substantially solid, amorphous film. Biologically active polypeptides stabilized in accordance with the embodiments are shown to retain significant biological activity even after extended storage conditions that would normally inactivate the polypeptides. Thus, the compositions offer significant advantages relative to previous formulations for biologically active polypeptides, such as antibodies (or antibody-enzyme conjugates), which typically require refrigeration or even freezing to maintain activity for any significant period of time. This allows previously highly unstable polypeptide compositions to be stored and transported over large distances with-out the typically required cold chain. Moreover, because the films of the embodiments can be rapidly dissolved in an aqueous solution, they can be easily reconstituted for use as reagents (e.g., diagnostic reagents) or therapeutics. Another advantage of the provided compositions is that the composition fosters storage of biologically active polypeptides (e.g., antibody-based therapeutic and diagnostic products) at concentrations that largely exceed their solubility limits but which may be necessary for administration and/or use without compromising the physical stability and performance of the agent. This is a significant advantage of this technology over standard lyophilization and conventional liquid based formulations which are current state of the art.

Accordingly, in some aspects the present disclosure relates to biologically active polypeptide compositions that may be directly administered to a subject (or reconstituted in a buffer and administered). Thus, the compositions themselves can be administered via buccal, sublingual mucosa, oral, nasal, ocular and skin surface contact. In some embodiments, the disclosure also relates to methods for administration and preparation of such polypeptide compositions that have been solubilized in an aqueous buffer. For example, the films can be solubilized and then administered via injection to a subject.

As noted above, in some embodiments, the present disclosure provides a composition that can be hydrated with a sterile aqueous solution and administered by other routes of administration, also known to bypass metabolism/degradation in the gastrointestinal tract such as sublingually by placing a reconstituted solution under the tongue. The resulting solution can be administered by intravenous or intramuscular injection. The solution can also be placed in a suitable delivery device for administration to the nasal cavity or to the respiratory tract via inhalation.

In some embodiments, a therapeutic composition of the present disclosure may be made by contacting a biologically active polypeptide (e.g., an antibody) with an orally dissolving film, or optionally, mixing an antibody with one or more excipients (surfactants, sugars, starches, etc.) and contacting the film surface with the mixture. In some embodiments, the biologically active polypeptide (e.g., an antibody or the antibody mixture) is then allowed to dry on the film, which is then ready for administration (or reconstitution).

### A. Biologically Active Polypeptides

In some embodiments, the present disclosure provides a biologically active polypeptide composition comprised within an amorphous, substantially solid film. The sold film also comprises a sugar and amphipathic surfactant. The biologically active polypeptide may be an antibody, such as a monoclonal antibody or a humanized antibody, an enzyme, a ligand, or a receptor. The receptor may comprise the extracellular domain of a receptor, such as a soluble form of a receptor. The antibody may comprise a full-length antibody, a heavy chain polypeptide, a light chain polypeptide, an Fab domain, or a single chain variable fragment (ScFv) polypeptide. In further aspects, the present disclosure provides a therapeutic composition comprising a fully dissolvable film and at least one antibody or a combination of several antibodies capable of binding distinctly different antigens.

In some embodiments the amount of a biologically active polypeptide placed on or incorporated into the film will fall within the range of 0.001 - 0.01 mg/mm³ or 0.01 - 0.1 mg/mm³ or 0.2-0.5 mg/mm³ depending upon the types of excipients utilized and the physical properties of the antibody itself.

The antibody may be a humanized antibody, a chimeric antibody, an antibody fragment, a bispecific antibody or a single chain antibody. An antibody as disclosed herein includes an antibody fragment, such as, but not limited to, Fab, Fab' and F(ab')2, Fd, single-chain Fvs (scFv), single-chain antibodies, disulfide-linked Fvs (sdfv) and fragments including either a VL or VH domain. In some aspects, the antibody or fragment thereof specifically binds epidermal growth factor receptor (EGFR1, Erb-B1), HER2/neu (Erb-B2), CD20, Vascular endothelial growth factor (VEGF), insulin-like growth factor receptor (IGF-1R), TRAIL-receptor, epithelial cell adhesion molecule, carcino-embryonic antigen, Prostate-specific membrane antigen, Mucin-1, CD30, CD33, or CD40.

Examples of monoclonal antibodies that may be comprised in the compositions provided herein include, without limitation, trastuzumab (anti-HER2/neu antibody); Pertuzumab (anti-HER2 mAb); cetuximab (chimeric monoclonal antibody to epidermal growth factor receptor EGFR); panitumumab (anti-EGFR antibody); nimotuzumab (anti-EGFR antibody); Zalutumumab (anti-EGFR mAb); Necitumumab (anti-EGFR mAb); MDX-210 (humanized anti-HER-2 bispecific antibody); MDX-210 (humanized anti-HER-2 bispecific antibody); MDX-447 (humanized anti-EGF receptor bispecific antibody); Rituximab (chimeric murine/human anti-CD20 mAb); Obinutuzumab (anti-CD20 mAb); Ofatumumab (anti-CD20 mAb); Tositumumab-I131 (anti-CD20 mAb); Ibritumomab tiuxetan (anti-CD20 mAb); Bevacizumab (anti-VEGF mAb); Ramucirumab (anti-VEGFR2 mAb); Ranibizumab (anti-VEGF mAb); Aflibercept (extracellular domains of VEGFR1 and VEGFR2 fused to IgG1 Fc); AMG386 (angiopoietin-1 and -2 binding peptide fused to IgG1 Fc); Dalotuzumab (anti-IGF-1R mAb); Gemtuzumab ozogamicin (anti-CD33 mAb); Alemtuzumab (anti-Campath-1/CD52 mAb); Brentuximab vedotin (anti-CD30 mAb); Catumaxomab (bispecific mAb that targets epithelial cell adhesion molecule and CD3); Naptumomab (anti-5T4 mAb); Girentuximab (anti-Carbonic anhydrase ix); or Farletuzumab (anti-folate receptor). Other examples include antibodies such as Panorex^{™} (17-1A) (murine monoclonal antibody); Panorex ((17-1A) (chimeric murine monoclonal antibody); BEC2 (ami-idiotypic mAb, mimics the GD epitope) (with BCG); Oncolym (Lym-1 monoclonal antibody); SMART M195 Ab, humanized 13' 1 LYM-1 (Oncolym), Ovarex (B43.13, anti-idiotypic mouse mAb); 3622W94 mAb that binds to EGP40 (17-1A) pancarcinoma antigen on adenocarcinomas; Zenapax (SMART Anti-Tac (IL-2 receptor); SMART M195 Ab, humanized Ab, humanized); NovoMAb-G2 (pancarcinoma specific Ab); TNT (chimeric mAb to histone antigens); TNT (chimeric mAb to histone antigens); Gliomab-H (Monoclonals-Humanized Abs); GNI-250 Mab; EMD-72000 (chimeric-EGF antagonist); LymphoCide (humanized IL.L.2 antibody); and MDX-260 bispecific, targets GD-2, ANA Ab, SMART IDIO Ab, SMART ABL 364 Ab or ImmuRAIT-CEA. Examples of antibodies include those disclosed in U.S. Pat. No. 5,736,167, U.S. Pat. No. 7,060,808, and U.S. Pat. No. 5,821,337.

Further examples of antibodies include Zanulimumab (anti-CD4 mAb), Keliximab (anti-CD4 mAb); Ipilimumab (MDX-101; anti-CTLA-4 mAb); Tremilimumab (anti-CTLA-4 mAb); (Daclizumab (anti-CD25/IL-2R mAb); Basiliximab (anti-CD25/IL-2R mAb); MDX-1106 (anti-PD1 mAb); antibody to GITR; GC1008 (anti-TGF-β antibody); metelimumab/CAT-192 (anti-TGF-β antibody); lerdelimumab/CAT-152 (anti-TGF-β antibody); ID11 (anti-TGF-β antibody); Denosumab (anti-RANKL mAb); BMS-663513 (humanized anti-4-1BB mAb); SGN-40 (humanized anti-CD40 mAb); CP870,893 (human anti-CD40 mAb); Infliximab (chimeric anti-TNF mAb; Adalimumab (human anti-TNF mAb); Certolizumab (humanized Fab anti-TNF); Golimumab (anti-TNF); Etanercept (Extracellular domain of TNFR fused to IgG1 Fc); Belatacept (Extracellular domain of CTLA-4 fused to Fc); Abatacept (Extracellular domain of CTLA-4 fused to Fc); Belimumab (anti-B Lymphocyte stimulator); Muromonab-CD3 (anti-CD3 mAb); Otelixizumab (anti-CD3 mAb); Teplizumab (anti-CD3 mAb); Tocilizumab (anti-IL6R mAb); REGN88 (anti-IL6R mAb); Ustekinumab (anti-IL-12/23 mAb); Briakinumab (anti-IL-12/23 mAb); Natalizumab (anti-α4 integrin); Vedolizumab (anti-α4 β7 integrin mAb); T1 h (anti-CD6 mAb); Epratuzumab (anti-CD22 mAb); Efalizumab (anti-CD11a mAb); and Atacicept (extracellular domain of transmembrane activator and calcium-modulating ligand interactor fused with Fc).

Other exemplary polypeptides include, but not limited to insulin, insulin-like growth factor, human growth hormone (hGH), tissue plasminogen activator (tPA), cytokines, such as interleukins (IL), e.g., IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosis factor (TNF), such as TNF alpha and TNF beta, TNF gamma, TNF-related apoptosis-inducing ligand (TRAIL); granulocyte colony-stimulating factor (G-CSF), granulocyte-macrophage colony-stimulating factor (GM-CSF), macrophage colony-stimulating factor (M-CSF), monocyte chemotactic protein-1 (MCP-1), vascular endothelial growth factor (VEGF), erythropoietin, or any other hormone growth factors. Additional suitable biologically active polypeptides include, but are not limited to, amylin, salmon-derived calcitonin (s-CT), glucagon-like peptide 1 (GLP-1), glucagon, parthyroid hormone (PTH1), oxytocin, desmopressin (8 D-Arg vasopressin), insulin, protein YY (PYY), cytokines and lymphokines such as IFNα, IFNβ, IFNγ.

The biologically active polypeptide, peptide or protein which can be used in the present disclosure includes but is not limited to antibiotics, hematopoietics, antiinfective agents, antidementia agents, antiviral agents, antitumoral agents, antipyretics, analgesics, antiinflammatory agents, antiulcer agents, antiallergic agents, antidepressants, anticonvulsants, psychotropic agents, cardiotonics, antiarrythmic agents, vasodilators, antihypertensive agents such as hypotensive diuretics, antidiabetic agents, anticoagulants, cholesterol lowering agents, therapeutic agents for osteoporosis, and hormones (e.g., steroid hormones).

While specific examples of the polypeptide for use in accordance with this disclosure are mentioned below, this does not mean that other known peptides or proteins are excluded. These peptides or proteins may be naturally occurring, recombinant or chemically synthesized substances.

The following is a partial listing of such peptides or proteins: cytokines, peptide hormones, growth factors, factors acting on the cardiovascular system, cell adhesion factors, factors acting on the central and peripheral nervous systems, factors acting on humoral electrolytes and hemal organic substances, factors acting on bone and skeleton, factors acting on the gastrointestinal system, factors acting on the kidney and urinary organs, factors acting on the connective tissue and skin, factors acting on the sense organs, factors acting on the immune system, factors acting on the respiratory system, factors acting on the genital organs, and various enzymes.

In some embodiments, the polypeptides are cytokines, peptide hormones, growth factors, factors acting on the cardiovascular system, factors acting on the central and peripheral nervous systems, factors acting on humoral electrolytes and hemal organic substances, factors acting on bone and skeleton, factors acting on the gastrointestinal system, factors acting on the immune system, factors acting on the respiratory system, factors acting on the genital organs, and enzymes. The cytokines include tymphokines, monokines, and hematopoietic factors. The lymphokines include interferons (e.g. interferon-α, -β and -γ), and interleukins (e.g. interleukin 2 through 11). The monokines include interleukin-1, tumor necrosis factors (e.g. TNF-α and -β), and malignant leukocyte inhibitory factor (LIF). The hematopoietic factors include, among others, erythropoietin, granulocyte colony stimulating factor (G-CSF), granulocyte-macrophage stimulating factor (GM-CSF) and macrophage colony stimulating factor (M-CSF). As factors having hematopoietic activity, factors having thrombopoietic (proliferation) activity, such as a leukocyte proliferation factor preparation (Leucoprol, Morinaga Milk), thrombopoietin, platelet proliferation stimulating factor and megakaryocyte proliferation (stimulating) factor could also be used.

The factors acting on bone and skeleton include bone GLa peptide, parathyroid hormone and its active fragments (osteostatin), histone H4-related bone formation and proliferation peptide (OGP) and their muteins, derivatives and analogs thereof.

The growth factors include nerve growth factors (NGF, NGF-2/NT-3), epidermal growth factor (EGF), fibroblast growth factor (FGF), insulin-like growth factor (IGF), transforming growth factor (TGF), platelet-derived cell growth factor (PDGF), and hepatocyte growth factor (HGF).

Peptide hormones include insulin, growth hormone, luteinizing hormone-releasing hormone (LH-RH), adrenocorticotropic hormone (ACTH), amylin, oxytocin, luteinizing hormone and other factors acting on the genital organs and their derivatives, analogs and congeners. As analogs of said LH-RH, such known substances are described in U.S. Patent Nos. 4,008,209, 4,086,219, 4,124,577, 4,317,815 and 5,110,904.

The factors acting on the central and peripheral nervous systems include opioid peptides (e.g. enkepharins, endorphins, kyotorphins), neurotropic factor (NTF), calcitonin gene-related peptide (CGRP), thyroid hormone releasing hormone (TRH), and salts and derivatives of neurotensin.

### B. Polypeptide Compositions

In general, an amorphous solid suitable for use in the present disclosure should be dissolvable upon contact with an aqueous liquid, such as saliva, In some embodiments, amorphous solids suitable for use in the present disclosure may be formed from any sugar, sugar derivative or combination of sugars/derivatives so long as the sugar and/or derivative is prepared as a liquid solution at a concentration that allows it to flow freely when poured but also forms an amorphous phase at ambient temperatures on a physical surface that facilitates this process, such as aluminum or Teflon. Examples of suitable sugars may include, but are not limited to glucose, dextrose, fructose, lactose, maltose, xylose, sucrose, coin sugar syrup, sorbitol, hexitol, maltilol. xylitol, mannitol, melezitose. raffinose, and a combination thereof. While not being bound to any particular theory, it is believed that sugars minimize interaction of the polypeptide with water during storage and drying, in turn, preventing damage to the three dimensional shape of the polypeptide during the drying process and subsequent loss of efficacy. In some embodiments, an amorphous solid suitable for use in the present disclosure may have a thickness of about 0.005 micrometers to about 5 millimeters.

In a further aspect dissolvable films may comprise a water-soluble polymer including, but not limited to, carboxymethyl cellulose, carboxyvinyl polymers, high amylose starch, hydroxyethyl cellulose, hydroxypropyl cellulose, hydroxypropyl methylcellulose, methylmethacrylate copolymers, polyacrylic acid, polyvinyl alcohol, polyvinylpyrrolidone, pullulan, sodium alginate, and combinations of these.

As discussed above, in certain aspects, dissolvable films of the embodiments may further comprise one or more sugars, oils, polyalcohols, gums and edible organic acids. Examples of suitable sugars may include, but are not limited to glucose, dextrose, fructose, lactose, maltose, xylose, sucrose, corn sugar syrup, sorbitol, hexitol, maltilol, xylitol, mannitol, melezatose, trehalose and a combination thereof. Examples of suitable oils that be used in composition may be included, but are not limited to, eucalyptol, menthol, vacrol, thymol, methyl salicylate, verbenone, eugenol, gerianol, melaleuca, C. verum, anethole, Sinapis alba, Eucalyptus Globulus, Mentha piperita, Myroxylon pereirae combinations of these. Examples of suitable polyalcohols may include, but are not limited to, glycerol, polyethylene glycol, propylene glycol, polyvinyl alcohol and various combinations. Examples of gums that can be sed in composition may include but are not limited to gum arabic, gum ghatti, gum karaya, gum tragacanth, khaya and albizia gums, guar gum, locust bean gum, Abelmoschus gum, Albizia gum, locust bean gum, honey locust, tara, almond, cashew, neem and *Moringa oleifera* gums. Examples of suitable edible organic acids may include, but are not limited to, citric acid, malic acid, tartaric acid, fumaric acid, phosphoric acid, oxalic acid, ascorbic acid and a combination thereof.

In addition, in some embodiments, certain sugars may also function as a binder which may provide ''substance" to pharmaceutical preparations that contain small quantities of very potent medications for ease of handling/administration. They may also hold components together or promote binding to surfaces (like the film backing) to ease drug delivery and handling. Lastly, they may also contribute to the overall pharmaceutical elegance of a preparation of an amorphous solid of the embodiments.

In certain embodiments, the polypeptide compositions of the present disclosure also may comprise a water-soluble polymer including, but not limited to, carboxymethyl cellulose, carboxyvinyl polymers, high amylose starch, hydroxyethyl cellulose, hydroxypropyl cellulose, hydroxypropyl methylcellulose, methylmethacrylate copolymers, polyacrylic acid, polyvinyl alcohol, polyvinyl pyrrolidone, pullulan, sodium alginate, poly(lactic-co-glycolic acid), poly(ethylene) oxide, poly (hydroxy alkanoate) and a combination thereof.

Furthermore, in some embodiments, the polypeptide compositions of the present disclosure may further comprise one or more oils, polyalcohols, surfactants, permeability enhancers, and/or edible organic acids. Examples of suitable oils may include, but are not limited to, eucalyptol, menthol, vacrol, thymol, methyl salicylate, verbenone, eugenol, gerianol and a combination thereof. Examples of suitable polyalcohols may include, but are not limited to, glycerol, polyethylene glycol, propylene glycol, and a combination thereof. Examples of suitable edible organic acids may include, but are not limited to, citric acid, malic acid, tartaric acid, fumaric acid, phosphoric acid, oxalic acid, ascorbic acid and a combination thereof. Examples of suitable surfactants may include, but are not limited to, difunctional block copolymer surfactants terminating in primary hydroxyl groups, such as Pluronic^{®} F68 commercially available from BASF, poly(ethylene) glycol 3000, dodecyl-β-D-maltopyranoside, disodium PEG-4 cocamido MIPA-sulfosuccinate ("DMPS"), etc It is believed that certain surfactants may minimize interaction of the polypeptide with itself and other polypeptides. They may also be capable of weakening cell membranes without causing permanent damage and, through this mechanism, promote uptake of large particles though rugged biological membranes such as the buccal mucosa.

In certain preferred aspects, a polypeptide composition of the embodiments comprises a zwitterionic surfactant. In some embodiments, the zwitterionic surfactant is a surfactant molecule which contains a group which is capable of being positively charged and a group which is capable of being negatively charged. In some embodiments, both the positively charged and negatively charged groups are ionized at physiological pH such that the molecule has a net neutral charge. In some embodiments, the positively charged group comprises a protonated or quaternary ammonium. In some embodiments, the negatively charged group comprises a sulfate, a phosphate, or a carboxylate. The zwitterionic surfactant further comprises one or more lipid groups consisting essentially of an alkyl, cycloalkyl, or alkenyl groups. Preferably, the zwitterionic surfactant comprises one or more lipid groups consisting essentially of an alkyl, cycloalkyl, or alkenyl groups with a carbon chain of more than 12 carbon atoms. In some embodiments, the lipid group has a carbon chain of 12-30 carbon atoms. In some embodiments, the lipid group has a carbon chain of 12-24 carbon atoms. In some embodiments, the lipid group has from 12, 13, 14, 15, 16, 17, 18, 19, 20, 21, 22, 23, to 24 carbons, or any range derivable thereof. In some embodiments, the zwitterionic surfactant is a polymeric structure which contains multiple zwitterionic groups and multiple lipid groups on a central backbone. In some embodiments, the zwitterionic surfactant is a polymer which has from about 50 to about 200 repeating units wherein each repeating units comprises one positively charged group, one negatively charged group, and one lipid group. In some embodiments, the zwitterionic surfactant is a polymer which has a 75 to 150 repeating units. In some embodiments, the central backbone is an alkyl, polyethylene glycol, or polypropylene chain. In some embodiments, the central chain is an alkyl group.

Some non-limiting examples of zwitterionic surfactants include 3-(N,N-Dimethyltetradecylammonio)propanesulfonate (SB3-14), 3-(4-Heptyl)phenyl-3-hydroxypropyl)dimethylammoniopropanesulfonate (C7BzO), 3-(decyldimethylammonio) propanesulfonate inner salt (SB3-10), 3-(dodecyldimethylammonio) propanesulfonate inner salt (SB3-12), 3-(N,N-dimethyloctadecylammonio) propanesulfonate (SB3-18), 3-(N,N-dimethyl-octylammonio) propanesulfonate inner salt (SB3-8), 3-(N,N-dimethylpalmitylammonio) propanesulfonate (SB3-16), 3-[N,N-dimethyl(3-myristoylaminopropyl)ammonio]propane-sulfonate (ASB-14), CHAPS, CHAPSO, acetylated lecithin, alkyl(C12-30) dialkylamine-N-oxide apricotamidopropyl betaine, babassuamidopropyl betaine, behenyl betaine, bis 2-hydroxyethyl tallow glycinate, C12-14 alkyl dimethyl betaine, canolamidopropyl betaine, capric/caprylic amidopropyl betaine, capryloamidopropyl betaine, cetyl betaine, 3-[(Cocamidoethyl)dimethylammonio]-2-hydroxypropanesulfonate, 3-[(Cocamidoethyl)dimethyl-ammonio]propanesulfonate, cocamidopropyl betaine, cocamidopropyl dimethylamino-hydroxypropyl hydrolyzed collagen, N-[3-cocamido)-propyl]-N,N-dimethyl betaine, potassium salt, cocamidopropyl hydroxysultaine, cocamidopropyl sulfobetaine, cocaminobutyric acid, cocaminopropionic acid, cocoamphodipropionic acid, coco-betaine, cocodimethylammonium-3-sulfopropylbetaine, cocoiminodiglycinate, cocoiminodipropionate, coco/oleamidopropyl betaine, cocoyl sarcosinamide DEA, DEA-cocoamphodipropionate, dihydroxyethyl tallow glycinate, dimethicone propyl PG-betaine, N,N-dimethyl-N-lauric acid-amidopropyl-N-(3-sulfopropyl)-ammonium betaine, N,N-dimethyl-N-myristyl-N-(3-sulfopropyl)-ammonium betaine, N,N-dimethyl-N-palmityl-N-(3-sulfopropyl)-ammonium betaine, N,N-dimethyl-N-stearamidopropyl-N-(3-sulfopropyl)-ammonium betaine, N,N-dimethyl-N-stearyl-N-(3-sulfopropyl)-ammonium betaine, N,N-dimethyl-N-tallow-N-(3-sulfopropyl)-ammonium betaine, disodium caproamphodiacetate, disodium caproamphodipropionate, disodium capryloamphodiacetate, disodium capryloamphodipropionate, disodium cocoamphodiacetate, disodium cocoamphodipropionate, disodium isostearoamphodipropionate, disodium laureth-5 carboxyamphodiacetate, disodium lauriminodipropionate, disodium lauroamphodiacetate, disodium lauroamphodipropionate, disodium octyl b-iminodipropionate, disodium oleoamphodiacetate, disodium oleoamphodipropionate, disodium PPG-2-isodeceth-7 carboxyamphodiacetate, disodium soyamphodiacetate, disodium stearoamphodiacetate, disodium tallamphodipropionate, disodium tallowamphodiacetate, disodium tallowiminodipropionate, disodium wheatgermamphodiacetate, N,N-distearyl-N-methyl-N-(3-sulfopropyl)-ammonium betaine, erucamidopropyl hydroxysultaine, ethylhexyl dipropionate, ethyl hydroxymethyl oleyl oxazoline, ethyl PEG-15 cocamine sulfate, hydrogenated lecithin, hydrolyzed protein, isostearamidopropyl betaine, 3-[(Lauramidoethyl)dimethylammonio]-2-hydroxypropane-sulfonate, 3-[(Lauramidoethyl)dimethylammonio]propanesulfonate, lauramido-propyl betaine, lauramidopropyl dimethyl betaine, lauraminopropionic acid, lauroamphodipropionic acid, lauroyl lysine, lauryl betaine, lauryl hydroxysultaine, lauryl sultaine, linoleamidopropyl betaine, lysolecithin, milk lipid amidopropyl betaine, myristamidopropyl betaine, octyl dipropionate, octyliminodipropionate, n-octyl-N,N-dimethyl-3-ammonio-1-propanesulfonate, n-decyl-N,N-dimethyl-3-ammonio-1-propanesulfonate, n-dodecyl-N,N-dimethyl-3-ammonio-1-propane-sulfonate, n-tetradecyl-N,N-dimethyl-3-ammonio-1-propanesulfonate, n-hexadecyl-N,N-dimethyl-3-ammonio-1-propanesulfonate, n-octadecyl-N,N-dimethyl-3-ammonio-1-propane-sulfonate, oleamidopropyl betaine, oleyl betaine, 4,4(5H)-oxazoledimethanol, 2-(heptadecenyl) betaine, palmitamidopropyl betaine, palmitamine oxide, PMAL-C6, PMAL-C12, PMAL-C16, ricinoleamidopropyl betaine, ricinoleamidopropyl betaine/IPDI copolymer, sesamidopropyl betaine, sodium C12-15 alkoxypropyl iminodipropionate, sodium caproamphoacetate, sodium capryloamphoacetate, sodium capryloamphohydroxypropyl sulfonate, sodium capryloamphopropionate, sodium carboxymethyl tallow polypropylamine, sodium cocaminopropionate, sodium cocoamphoacetate, sodium cocoamphohydroxypropyl sulfonate, sodium cocoamphopropionate, sodium dicarboxyethyl cocophosphoethyl imidazoline, sodium hydrogenated tallow dimethyl glycinate, sodium isostearoamphopropionate, sodium lauriminodipropionate, sodium lauroamphoacetate, sodium oleoamphohydroxypropylsulfonate, sodium oleoamphopropionate, sodium stearoamphoacetate, sodium tallamphopropionate, soyamidopropyl betaine, stearyl betaine, 3-[(Stearamidoethyl)dimethylammonio]-2-hydroxypropanesulfonate, 3-[(Stearamidoethyl)-dimethylammonio]propanesulfonate, tallowamidopropyl hydroxysultaine, tallowamphopoly-carboxypropionic acid, trisodium lauroampho PG-acetate phosphate chloride, undecylenamidopropyl betaine, and wheat germamidopropyl betaine.

### C. Methods of Preparing Polypeptide Compositions

In one embodiment, a polypeptide composition comprising an amorphous solid may be made by preparing a solution comprising a sugar, sugar derivative or combination of sugars/derivatives in a buffer and optionally other additives previously mentioned. In some embodiments, a sugar, sugar derivative or combination of sugars/derivatives may be present in the solution in an amount up to about 60% by weight of the solution. In some embodiments, an additive may be present in an amount of about 5% or less by weight of the solution In general, the solution comprising the sugar, sugar derivative or combination of sugars/derivatives is made at a concentration higher than the desired final concentration to compensate for any dilution that may occur when the polypeptide is added. The desired polypeptide may be added to the solution at a concentration known to induce the desired response The mixture may then be stirred at ambient temperature until a substantially homogeneous mixture is obtained. In some embodiments, the mixture may then be briefly sonicated under cooled conditions, *e*.*g*. 4° C., to remove any air bubbles that may have developed. In other embodiments, the mixture may be slightly heated, *e.g*., heated to 40° C. or below, slightly cooled, and in some instances may be frozen. In some embodiments, a polypeptide composition of the present disclosure may be made without freeze drying or spray draying. The final formulation may then be cast onto a flat backing surface in a laminar flow hood and allowed to form an amorphous solid at ambient temperatures (15-20° C.). Examples of suitable backing surfaces may include, but are not limited to, thin layers of aluminum, Teflon, silicate, polyetheretherketone, low density polyethylene, ethyl cellulose, etc. Once the process is complete the polypeptide composition can be peeled from the backing and placed in the mouth for immunization purposes and/or stored at ambient temperature for up to three years from manufacture.

In another embodiment, a polypeptide composition of the present disclosure may be made by contacting an amorphous solid with an polypeptide, or optionally, mixing a polypeptide with one or more excipients (surfactants, sugars, starches, etc.) and contacting the amorphous solid with the mixture so as to dispose the polypeptide within the amorphous solid. In some embodiments, the mixture is then allowed to dry, which is then ready for administration.

In some embodiments, polypeptide compositions of the present disclosure may further comprise a protective layer disposed on a surface of an amorphous solid comprising a polypeptide. Exemplary protective layers may include, but are not limited to, an additional layer(s) of film, such as polyethylene, polyurethane, polyether etherketone, etc, and/or an additional layer(s) of an amorphous solid that does not contain any polypeptide.

The amount of polypeptide that may be used in a composition of the present disclosure may vary greatly depending upon the type of polypeptide used, the formulation used to prepare the composition, the size of the amorphous solid, the solubility of the polypeptide, etc. One of ordinary skill in the art with the benefit of this disclosure will be able to determine a suitable amount of polypeptide to include in a composition of the present disclosure.

It is also important to note that when formulating a polypeptide composition of the present disclosure one must also consider any toxicity and/or adverse effects. Furthermore, in an effort to create a stable polypeptide composition, it may also be important to identify a ratio of ingredients that interacts with water and the polypeptide in a manner that prevents crystallization during drying.

Any substantially solid surface can be used for casting and/or drying compositions of the embodiments. For example, the surface can be a polymer (*e*.*g*., plastic) or a metal surface. In some aspects, the surface has a low coefficient of friction (*e.g.,* a siliconized, non-stick surface) to provide easy removal of films. In some embodiments, a glass plate can be used for casting of the polypeptide compositions. Composition that have been cast on a surface can then be dried, for instance, under a controlled, laminar flow of air at room temperature, or under refrigerated conditions. Similarly, polypeptide compositions suitable for use in the present disclosure can be prepared in a single-layer or multi-layers.

In general, the polypeptide compositions of the present disclosure may be formulated so as to dissolve in a relatively short period of time, for example, from about 5 to 60 seconds or 1 to 30 minutes. When administered, a polypeptide composition of the present disclosure may be handled by a portion of the composition that does not contain a polypeptide and may be placed in the upper pouch of the cheek for buccal delivery, or far under the tongue for sublingual delivery or reconstituted and utilized as a solution for inhalation or as a nasal spray.

In certain aspects, methods are provided for producing polypeptide compositions in substantially solid carriers. Such a method may comprise obtaining or formulating a solution comprising sufficient stabilizers (e.g., sugars and sugar derivatives, polymers) and permeability enhancers (e.g., surfactants, such as a zwitterionic surfactant of the embodiments) in a solvent system (e.g., distilled deionized water, ethanol, methanol). In some cases, formulation is such that the total amount of solid components added to the solvent are within the concentration of 10% - 90% w/w. This suspension can be prepared by stirring, homogenization, mixing and/or blending these compounds with the solvent. In some cases, small portions of each component (~1/10 the total amount) are added to the solvent and the solution mixed before adding additional portions of the same agent or a new agent.

In certain aspects, once each stabilizer and permeability enhancer is added, the bulk solution is placed at 4°C for a period of time between 2-24 hours. In some aspects, the bulk solution is subjected additional homogenization, such as sonication (e.g., for a period of 5-60 minutes) to remove trapped air bubbles in the preparation. After sonication is complete, the polypeptide, such as an antibody, is added to the preparation. In some cases, the amount of polypeptide will range from of 0.1 - 30% of the total solid concentration.

In some cases, the preparation is then slowly piped into molds of a shape suitable for the application. The molds can be constructed of a variety of materials including, but not limited to, stainless steel, glass, silicone, polystyrene, polypropylene and other pharmaceutical grade plastics. In some cases, the preparation can be placed in the molds by slowly pouring by hand or by pushing the preparation through a narrow opening on a collective container at a slow controlled rate *(e.g.,* 0.25 ml/min) to prevent early hardening and/or bubble formation in the final film product. In certain preferred aspects, films will be poured to a thickness of 12.5 - 1000 µm. In some aspects, molds for casting of films will be sterilized by autoclaving and placed in laminar air flow hoods prior to casting.

In further aspects, molds may also be lined with a peelable backing material suitable for protection of the film product. Suitable backings include, without limitation, aluminum, gelatin, polyesters, polyethylene, polyvinyl and poly lactic co-glycolide polymers, wax paper and/or any other pharmaceutically acceptable plastic polymer.

In some cases, cast films will remain at ambient temperature (e.g., 20-25 °C), such as in a laminar flow hood for 2-24 hours after which time a thin, peelable film will be formed. In some cases, this film may be opaque or translucent. In some cases, individual films are peeled from the casting/drying surface, wrapped in wax paper and stored at room temperature in sealable plastic bags under controlled humidity conditions. However, in certain aspects, films can be stored at lower temperatures, such as at 4°C, under controlled humidity as well.

In certain aspects, multilayer films can also be created at this time by applying a second coating of as solution containing the same polypeptide as the first layer or another different polypeptide to the thin film. Again, in some cases, this will remain at ambient temperature (e.g., 20-25 °C), such as in a laminar flow hood, for an additional 2-24 hours after which time a thin, peelable film will be formed. Again the film may be opaque or translucent.

In certain cases, films will be dissolved in a solution prior to use. For example, water or warmed saline (e.g., ~37°C, body temperature) may be used. In some cases, the resulting solution can be screened for enzyme activity to determine the effectiveness of the formulation to retain the potency of the preparation over time.

### D. Methods of Treatment

A number of therapeutic methods can be employed with the polypeptide compositions of the present disclosure, including therapeutic methods for diabetes control, glycemic control, treatment of obesity (e.g., through induction of satiety or other methods), treatment of cancer, treatment of infections, and treatment of psychiatric and seizure disorders. In addition, a wide variety of direct and indirect biological activities associated with the various polypeptides can be utilized for a wide range of various treatments or therapies; such therapies include, but are not limited to, antibiotic therapies, hematopoietic therapies, antiallergic therapies, hormone therapies, polypeptide supplement therapies, diagnostic assays, antidepressants and psychotropic therapies, antitumor therapies, antiarrhythmic therapies, vasodilator therapies, vasoconstrictor therapies, antihypertensive therapies, diabetes prevention and control, anticoagulant therapies, appetite suppressant activities, blood glucose control, glycemic control, satiety, anti-infective therapies, and osteoporotic therapies. These treatments or therapies could be accomplished upon successful transport of the appropriate polypeptide across the oral mucosa and into the bloodstream of the recipient.

Also provided herein are methods for treating or preventing a symptom of diseases characterized by missing or aberrant protein activity, by replacing the missing protein activity or overcoming the aberrant protein activity. Diseases characterized by dysfunctional or aberrant protein activity include, but are not limited to, cancer and proliferative diseases, genetic diseases (e.g., cystic fibrosis), autoimmune diseases, diabetes, neurodegenerative diseases, cardiovascular diseases, and metabolic diseases. The present disclosure provides a method for treating such conditions or diseases in a subject by introducing the protein using the compositions provided herein, wherein the biologically active polypeptide encodes for a protein that antagonizes or otherwise overcomes the aberrant protein activity present in the cell of the subject. Specific examples of a dysfunctional protein are the missense mutation variants of the cystic fibrosis transmembrane conductance regulator (CFTR) gene, which produce a dysfunctional protein variant of CFTR protein, which causes cystic fibrosis.

### II. Definitions

The term "polypeptide" is used in its broadest sense to refer to a compound of two or more subunit amino acids, amino acid analogs, or peptidomimetics. The subunits may be linked by peptide bonds. In another embodiment, the subunit may be linked by other bonds, e.g. ester, ether, etc. As used herein the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is commonly called a polypeptide or a protein.

The term "therapeutic agent" refers to any agent that, when administered to a subject, has a therapeutic, diagnostic, and/or prophylactic effect and/or elicits a desired biological and/or pharmacological effect.

As used herein, the phrase "biologically active" refers to a characteristic of any substance that has activity in a biological system and/or organism. For instance, a substance that, when administered to an organism, has a biological effect on that organism, is considered to be biologically active.

As used herein, the phrase "biologically active polypeptide" is meant to refer to polypeptides, peptides and proteins, and refers to a polymeric form of amino acids of any length, which can include coded and non-coded amino acids, chemically, or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like. Biologically active polypeptides useful in the present invention include, without limitation, cytokines, growth factors, hematopoietic factors, hormones, enzymes, antibodies and the like. Biologically active polypeptides share the biological activity associated with the base polypeptide. Biologically active polypeptides can be any length, e.g., from about 5 to about 20 amino acids; from about 10 to about 60 amino acids; from about 25 to about 75 amino acids; from about 50 to about 150 amino acids; from about 75 to about 250 amino acids; from about 100 to about 400 amino acids; from about 200 to about 600 amino acids, and larger.

As used herein, the term "subject" or "patient" refers to any organism to which a composition in accordance with the disclosure may be administered, e.g., for experimental, diagnostic, prophylactic, and/or therapeutic purposes. Typical subjects include animals (e.g., mammals such as mice, rats, rabbits, non-human primates, and humans) and/or plants.

As used herein, the term "therapeutically effective amount" means an amount of an agent to be delivered (e.g., nucleic acid, drug, therapeutic agent, diagnostic agent, prophylactic agent, etc.) that is sufficient, when administered to a subject suffering from or susceptible to a disease, disorder, and/or condition, to treat, improve symptoms of, diagnose, prevent, and/or delay the onset of the disease, disorder, and/or condition.

As used herein, the term "treating" refers to partially or completely alleviating, ameliorating, improving, relieving, delaying onset of, inhibiting progression of, reducing severity of, and/or reducing incidence of one or more symptoms, features, or clinical manifestations of a particular disease, disorder, and/or condition. For example, "treating" cancer may refer to inhibiting survival, growth, and/or spread of a tumor. Treatment may be administered to a subject who does not exhibit signs of a disease, disorder, and/or condition (e.g., prior to an identifiable disease, disorder, and/or condition), and/or to a subject who exhibits only early signs of a disease, disorder, and/or condition for the purpose of decreasing the risk of developing pathology associated with the disease, disorder, and/or condition. In some embodiments, treatment comprises delivery of a protein to a subject in need thereof.

### IV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Characterization of Polypeptide Compositions

Various formulations preserved antibody and enzymatic activity after drying and storage in thin film matrix. A horseradish peroxidase conjugated goat anti-mouse IgG secondary antibody was mixed with a variety of formulations and dried in a thin film matrix. Films were stored at 25°C for 48 hours prior to rehydration. Each solution was then utilized in a Western blot assay to detect murine beta-actin in cellular lysates prepared from murine hepatocytes. Data is reported as percent recovery of activity (FIG. 1). This was calculated by comparing band intensity of blots produced from antibody dried in each formulation with blots generated by stock antibody stored in the supplier's buffer at 4°C. Formulations evaluated were as follows: (1) 0.5% w/w HPMC/2% w/v sorbitol, (2) 1.5% w/w HPMC/2% v/v glycerol, (3) 1 M melezitose/5% PEG 3000, (4) 3% w/w HPMC/2% w/v sorbitol, (6) 5% polyvinylpyrrolidone (mw 40,000). Each film also contained 0.2% w/v tragacanth gum and were buffered in 10 mM Tromethamine USP, pH 8.0. The drying process was complete within 6 hours.

Film technology not only stabilizes antibodies such that they can effectively bind to an antigen after long-term storage at room temperature but it also maintains the activity of a recombinant enzyme conjugated to an antibody allowing it to be detected in a diagnostic or therapeutic application. This is exemplified in FIG. 2A where the activity of a horseradish peroxidase enzyme conjugated to a goat anti-mouse IgG secondary antibody was high when it was stabilized in film matrix comprising of 1.5% w/w HPMC/2% w/v sorbitol/0.2% w/v tragacanth gum in 100 mM phosphate buffered saline pH 7.4 and stored at 20°C for 3 months prior to reconstitution. The ability of the antibody to bind to a primary antibody against human beta-actin and the activity of the peroxidase conjugate to develop single protein bands on Western blots of lysates collected from human hepatocytes were not compromised during the drying process and subsequent storage. The graph in FIG. 2B depicts similar band densities generated for antibody dried in film matrix and stored at 20 °C for 90 days and standard stock stored at 4°C. Antibodies were heated to 65 °C for 30 minutes prior to use in Western Blot. (Boil = Negative Control; RT= Band Density for antibody stock from manufacturer stored at room temperature for 24 hours.)

The presence of antibody was also found to influence the moisture retained in dried film formulations. Moisture content for films of uniform weight containing 10 micrograms of a monoclonal antibody (ANTIBODY) /mm³ film and blank controls was assessed by Karl Fischer titration according to USP standards. The results are shown in FIG. 3. While formulations alone (blank) did not significantly vary in moisture content after drying was complete, they did influence the ability of the embedded antibody to retain moisture in the dried state to varying degrees. Formulations evaluated were as follows: (1) 0.5% w/w HPMC, (2) 0.5% w/w HPMC/2% w/v sorbitol, (3) 0.5% w/w HPMC/2% v/v glycerol, (4) 1.5% w/w HPMC/2% v/v glycerol, (5) 3% w/w HPMC/2% w/v sorbitol. All films contained 0.2% w/v tragacanth gum and were buffered in 10 mM Tromethamine USP, pH 8.0. The drying process for all films was complete within 5 hours.

In addition, antibody was shown to significantly impact dissolution rate of films in simulated saliva. Films of uniform weight containing 50 micrograms of a monoclonal antibody (ANTIBODY) per mm³ of film and blank controls were placed in simulated human salivary fluid at 37 °C under gentle stirring. Dissolution rate was calculated by dividing the starting weight of the film by the time at which the film could no longer be visibly detected in the solvent. The results are shown in FIG. 4. Formulations alone (blank) provided a variety of dissolution rates after drying was complete. These differences were enhanced in the presence of antibody. Formulations evaluated were as follows: (1) 0.5% w/w HPMC/2% w/v sorbitol, (2) 0.5% w/w HPMC/2% w/v glycerol, (3) 1.5% w/w HPMC/2% w/v sorbitol, (4) 1.5% w/w HPMC/2% v/v glycerol, (5) 3% w/w HPMC/2% w/v sorbitol, (6) 3% HPMC/2% v/v glycerol. Each film contained 0.2% w/v tragacanth gum and was buffered in 100 mM phosphate buffered saline, pH 7.4. Drying for all films was complete in 4 hours. Simulated human saliva fluid consisted of: KCl 0.15 g/L, NaCl 0.12 g/L, Sodium Bicarbonate 2.1 g/L, alpha-amylase 2.0 g/L and gastric mucin 1.0 g/L as described in Davis *et al.* 1971, incorporated herein by reference.

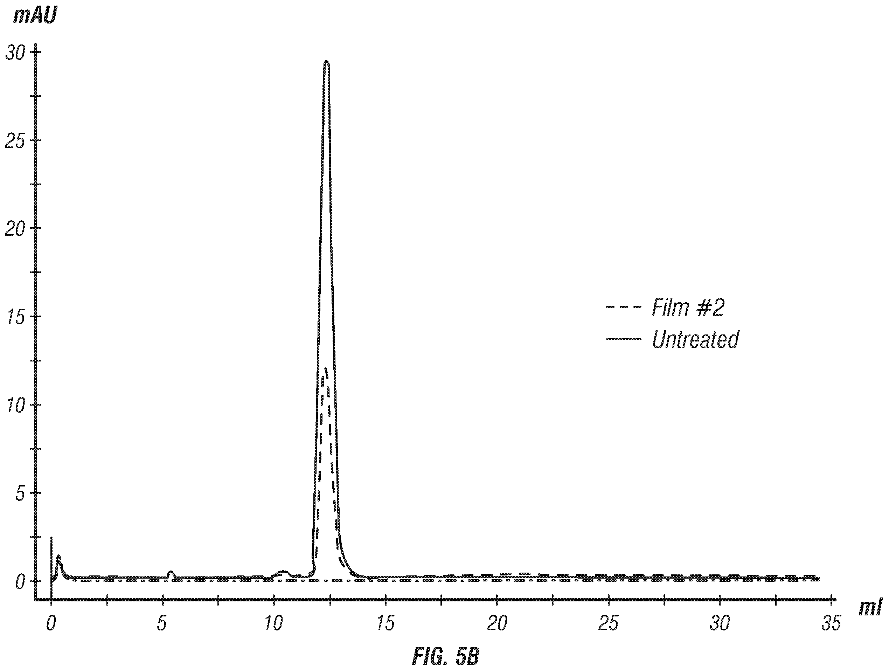

To evaluate the binding of affinity of Human IgG1 Anti-Pertusis Toxin Antibody (hu11E6) stabilized in film matrices, hu11E6 was added to 1.5% HPMC, 0.2% tragacanth gum, 2% sorbitol and PMAL C16 (1%), pH 8.1 and dried in a thin film matrix at 20°C for 14 hours. Once drying was complete, films were reconstituted with sterile water for injection. An indirect ELISA comparing binding affinities of unformulated hu11E6 (untreated) and upon reconstitution of dried film (Film 1, 2) was performed as described in Nguyen *et al.,* 2015, incorporated herein by reference (FIG. 5A). Stabilization in the films preserved the binding affinity of the hu11E6 antibody. To evaluate whether antibodies aggregate in the film matrix, purified hu11E6 (untreated) and hu11E6 after reconstitution from the thin film matrix were evaluated by size exclusion chromatography using an ActaPure S200 system with a 100 µl loop (FIG 5B). Both the reconstituted antibody and the untreated antibody resolved as a single peak, indicating the absence of aggregates in the final product.

To evaluate whether a variety of film formulations preserved the binding affinity of primary antibodies in an ELISA assay. A monoclonal mouse anti-human alpha-1 antitrypsin antibody (178260 Millipore) was mixed with three different formulations of film. Formulation 1: 3% HPMC, 0.2% tragacanth gum, 10% trehalose. Formulation 2: 1.5% HPMC, 0.2% tragacanth gum, 5% sucrose, 1% PEG 3000. Formulation 3: 1.5% HPMC, 0.2% tragacanth gum, 2% sorbitol, 1% PMAL C16. Each formulation was prepared in 10mM Tris, pH 8.1. Films were dried under ambient conditions overnight and reconstituted the following day. Reconstituted solutions were then utilized in an A1AT ELISA (Le *et al.,* 2005). Each formulation showed excellent recovery of binding affinity of 178260 for alpha-1 antitrypsin upon reconstitution of thin film matrix (FIG. 6A). Percent recovery is the relative absorbance reading generated by an assay utilizing 178260 from reconstituted films with that of fresh 178260 as supplied by the manufacturer for each given concentration of A1AT standard. Data reflect the results from 3 separate experiments including triplicate samples for each formulation. A standard curve was generated with fresh 178260 (Fresh stock) and antibody stabilized in a thin film matrix (Form 3) (FIG. 6B).

To assess antibody stability after storage, antibodies stored for 30 days at room temperature in a film formulation was evaluated by ELISA. A monoclonal mouse anti-human alpha-1 antitrypsin antibody (178260 Millipore) was mixed with a formulation containing 1.5% HPMC, 0.2% tragacanth gum, 2% sorbitol and 10mg/ml PMAL C16. Films were dried under ambient conditions overnight and stored at 20°C for 30 days. Reconstituted solutions were utilized in an A1AT ELISA. Recovery of binding affinity of 178260 for alpha-1 antitrypsin upon reconstitution was found to be nearly 100% following storage in film formulation (FIG. 7A). Percent recovery is the relative absorbance reading generated by an assay utilizing 178260 from reconstituted films with that of fresh 178260 as supplied by the manufacturer for each given concentration of A1AT standard. The antibodies stored in the film formulation were compared with manufacturer's stock stored at RT (instead of 4 °C as recommended), and found to have drastically increased percent recovery. Data reflect the results from 3 separate experiments. A standard curve was generated with fresh 178260, antibody stored at RT in film for 30 days and stock stored at RT (FIG. 7B). The absorbance observed in the ELISA assay for the antibodies stored in film are very similar to the fresh antibodies, indicating that binding affinity of primary antibodies is preserved extremely well after 30 days of storage in the HPMC, tragacanth gum, sorbitol and HPMC film.

To evaluate whether horseradish peroxidase activity would be affected by dilution in several formulations designed to stabilize biologicals, an ELISA was performed. A polyclonal donkey horseradish peroxidase conjugated anti-mouse IgG antibody (AP192P EMD Millipore) was mixed with three different formulations developed to stabilize biologicals in a thin film matrix. Formulation 1: 3% HPMC, 0.2% tragacanth gum, 20% sucrose in 100 mM phosphate buffered saline (pH 7.4). Formulation 2: 1.5% HPMC, 0.2% tragacanth gum, 5% sucrose, 2% PEG 5000 in 10 mM Tris buffer (pH 8.1). Formulation 3: 1.5% HPMC, 0.2% tragacanth gum , 10% melezitose in 50 mM citrate buffer (pH 5.5). These preparations were utilized in an A1AT ELISA to determine if excipients would interfere with the readout of the assay (FIG. 8A). Binding affinity of AP192P for primary antibody utilized in the ELISA assay is shown as percent relative to the control, fresh AP192P (FIG. 8A). Data reflect the results from 3 separate experiments including triplicate samples for each formulation. A standard curve was generated with fresh AP192P (fresh stock) and antibody in formulation 2, showing that these film technologies do not impact performance of a horseradish peroxidase conjugated secondary antibody in the ELISA assay (FIG. 8B).

Since the formulations designed to stabilize biologicals had no negative effect on the activity of the HRP-conjugated secondary antibodies, several were evaluated as substrates for storage of the antibodies. AP192P was mixed with three different formulations. Formulation 1: 3% HPMC, 0.2% tragacanth gum, 40 mg/ml mannitol, 10 mg/ml sucrose and 10 mg/ml PMAL C16 in 100 mM phosphate buffered saline (pH 7.4). Formulation 2: 1.5% HPMC, 0.2% tragacanth gum , 10% melezitose in 50 mM citrate buffer (pH 5.5). Formulation 3: 1.5% HPMC, 0.2% tragacanth gum, 2% sorbitol, 1% PMAL C16 in 10mM Tris buffer (pH 8.1). Films were dried under ambient conditions overnight and reconstituted the following day. Reconstituted solutions were utilized in an A1AT ELISA. Binding affinity of AP192P for a primary mouse anti-human alpha-1 antitrypsin antibody (178260) was recovered to nearly 100% upon reconstitution of the thin film matrix of form 3, while formulation 1 yielded antibodies with close to 80% activity (FIG. 9A). Percent recovery is the relative absorbance reading generated by an assay utilizing AP192P from reconstituted films with that of fresh AP192P as supplied by the manufacturer for each given concentration of A1AT standard. Data reflect the results from 3 separate experiments including triplicate samples for each formulation. A standard curve was generated with fresh AP192P (fresh) and antibody stabilized in 2 different thin film matrices (Form 1 and 3) (FIG. 9B). It can be seen that the antibodies stored in film formulation 3 track extremely well with the fresh antibodies, indicating that antibodies stored in the film are not negatively impacted by ambient temperatures.

As drying the secondary antibodies in 1.5% HPMC, 0.2% tragacanth gum, 2% sorbitol and 10 mg/ml PMAL C16 formula did not have any negative effect on the binding affinity of the antibodies, the same formulation was evaluated for longer term storage (30 days). AP192P was mixed with a formulation containing 1.5% HPMC, 0.2% tragacanth gum, 2% sorbitol and 10mg/ml PMAL C16 in 10mM Tris buffer (pH 7.4). Films were dried under ambient conditions overnight and stored at 20°C for 30 days. Reconstituted solutions were then utilized in an A1AT ELISA (FIG. 10A). Absorbance readings were generated by an assay utilizing AP192P from reconstituted films with that of fresh AP192P as supplied by the manufacturer for each given concentration of A1AT standard. Recovery of binding affinity of reconstituted AP192P for a primary mouse anti-human alpha-1 antitrypsin antibody is shown as percent recovery relative to fresh antibody (FIG. 10A). Results were also compared with manufacturer's stock stored at RT (instead of -20 °C as recommended) (FIG. 10A). Data reflect the results from 3 separate experiments. A representative standard curve generated with fresh AP192P, antibody stored at RT in film for 30 days and stock stored at RT (FIG. 10B). It is clear from these experiments that storage in the film stabilizes the antibodies and results in significantly greater retention of activity than in the manufacturer's buffer.

To evaluate whether enzymatic activity was disturbed during the film forming process, streptokinase activity was examined. Streptokinase C, isolated from the Streptococcus species (Sigma S0827), at a dose of 4 International Units (IU) was mixed with 3 different formulations and dried at room temperature for 16 hours. Formulation 1: 1.5% HPMC, 0.2% tragacanth gum, 5% sucrose, PMAL C16 (1%) in 0.1 M phosphate buffered saline (pH 7.4). Formulation 2: 3% HPMC, 0.2% tragacanth gum, 2% sorbitol, PMAL C16 (1%) in 0.1M citrate buffer (pH 5.5). Formulation 3: 1.5% HPMC, 0.2% tragacanth gum, 5% trehalose, PMAL C16 (1%) in 10 mM Tris buffer (pH 8.1). Once drying was complete, the films were then reconstituted with a buffer consisting of 10 mM Tris (pH 7.4), 0.1M NaCl and human albumin (1 mg/ml). Streptokinase activity present in each reconstituted film was measured using a standard chromogenic assay (FIG. 11). Percent potency is the activity of streptokinase in the film with respect to freshly thawed enzyme measured in the same assay plate. Data reflect the average streptokinase activity obtained from six individual films for each formulation. Error bars reflect the standard deviation of the data. As the percent potency of Streptokinase C was about 100% for enzyme stored in each formulation, it is clear that enzymatic activity was no disturbed during the film forming process.

One concern regarding the storage of enzymes is whether increasing concentrations of enzymes impact their stability. To determine whether the amount of streptokinase incorporated into a film impacts its stability, several different amounts of Streptokinase C were placed in a formulation consisting of 1.5% HPMC, 0.2% tragacanth gum, 2% sorbitol, PMAL C16 (1%) in 10 mM Tris buffer (pH 8.1). The activity of streptokinase in the preparation was determined prior to drying by a standard chromogenic assay. Aliquots of the preparation were placed in unit dose molds and dried at 20°C for 17 hours. Once drying was complete, films were stored in sealed pouches at 25°C for 15 days. At that time, films were reconstituted with a buffer consisting of 10 mM Tris (pH 7.4), 0.1M NaCl and human albumin (1 mg/ml) and streptokinase activity present in each reconstituted film measured. Percent potency is the activity of streptokinase in the film after 15 days of storage with respect to activity measured in the formulation prior to drying (FIG 12). Data reflect the average streptokinase activity obtained from eight individual films for each amount of streptokinase indicated below. Error bars reflect the standard deviation of the data. After 15 days of storage, about 100% of streptokinase activity was recovered for each concentration, showing that the amount of enzyme incorporated into the film does not significantly impact its stability. It is also important to note that streptokinase activity could not be detected in manufacturer's samples (i.e. enzyme in phosphate buffer alone, pH 7.5) stored under the same conditions (data not shown).

To further provide further evidence that these formulations do not impact enzymatic activity, Beta-Galactosidase activity was tested. Various amounts of β-Galactosidase from Escherichia coli (Grade VIII, 500 Units/mg protein) were mixed with 3 different formulations developed to stabilize biologicals in a thin film matrix or with 50mM Tris, pH 7.3. Formulation 1: 1.5% HPMC, 0.2% tragacanth gum, 2% sorbitol, 1% PMAL C16 in 100mM phosphate buffered saline (pH 7.4). Formulation 2: 1.5% HPMC, 0.2% tragacanth gum, 2% sorbitol, 1% PMAL C16 in 50mM citrate buffer (pH 5.5). Formulation 3: 1.5% HPMC, 0.2% tragacanth gum, 2% sorbitol, 1% PMAL C16 in 10 mM Tris buffered saline (pH 7.8) The enzymatic activity present in each formulation was assessed with a Galacto Light Plus Detection System (Applied Biosystems) and is shown in FIG. 13. The data reflect the results from 3 separate experiments including triplicate samples for each formulation. Performance of the enzyme was not significantly impacted by any of the formulations tested.

Film technology was also found to preserve enzymatic activity after storage at 25°C for 10 days. Recombinant beta-galactosidase (MP Biomedicals, 10 mg, 300 units/mg) was incorporated into film matrix (3% HPMC/2% glycerol) in 10 mM phosphate buffered saline pH 7.0, dried under ambient conditions and stored at room temperature for 10 days. Films were then reconstituted and added to a solution consisting of 4 mg/ml *o*-nitrophenyl-β-D-galactoside (ONPG), 1mM MgCl₂ and 50 mM beta-mercaptoethanol (pH 7). Samples were placed in a plate reader and absorbance at 420 nm recorded every 20 seconds for 2 minutes. The results are shown in FIG. 14. When the enzyme was stored in phosphate buffer (pH 7.4) alone at room temperature (LIQUID RT), very little enzymatic activity was detected at the 10 day time point. This is not surprising, since the manufacturer states that the enzyme will remain active for only 20 hours under these conditions. Beta-galactosidase dried in buffer and 1.5% HPMC (FILM no FORM) demonstrated more activity than the liquid formulation, however this was significantly lower than what was seen from antibody stored frozen as recommended by the manufacturer (FRESH -20 °C Storage). Reaction rates for each enzyme are summarized in Table 1 below.

**Table 1. Calculated Reaction Rates for Beta-Galactosidase Stabilized in Film Formulations and Stored at 25°C for 10 Days.**

| **Condition** | **Reaction Rate (µmol/min)** |
|---|---|
| Liquid RT | 0.00029 |
| Film No Form | 0.0012 |
| Film Form | 0.1069 |
| Fresh (-20 °C storage) | 0.0204 |

To evaluate whether films formed with Beta-Galactosidase were stable for longer periods of time, they were evaluated after 30 days of storage at room temperature. Various amounts of β-Galactosidase from Escherichia coli (Grade VIII, 500 Units/mg protein) were mixed with 2 different formulations. Formulation 1: 3% HPMC, 0.2% tragacanth gum, 5% melezitose, 1% PEG 3000 in 100mM phosphate buffered saline (pH 7.4). Formulation 2: 1.5% HPMC, 0.2% tragacanth gum, 2% sorbitol, 1% PMAL C16 in 10mM Tris buffer (pH 8.1). The films were dried at ambient temperature for 16 hours and then stored at 20 °C for 30 days prior to reconstitution. The enzymatic activity present in each reconstituted film was assessed with a Galacto Light Plus Detection System (Applied Biosystems) (FIG. 15). Data reflect the results from 3 separate experiments including triplicate samples for each formulation. Both formulations allowed for significant Beta-Galactosidase activity after reconstitution, with very high percentages of activity remaining after storage.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references, to the extent that they provide exemplary procedural or other details supplementary to those set forth herein, are specifically incorporated herein by reference.
Davis et al., J. Pharm. Sci. 60(3):429-432, 1971.
Le et al., J Control Release. 108(1):161-77, 2005.
Mahboubi et al., Iran JPharm Res. 11(4): 1087-93, 2012.
Nguyen et al., Sci. Transl. Med. 7(316):316ra195. doi: 10.1126/scitranslmed.aad0966, 2015.
Sands et al., J Thromb Haemost. 2(8):1411-1415, 2004.
U.S. Patent No. 4,008,209
U.S. Patent No. 4,086,219
U.S. Patent No. 4,124,577
U.S. Patent No. 4,317,815
U.S. Patent No. 5,110,904
U.S. Patent No. 5,736,167
U.S. Patent No. 5,821,337
U.S. Patent No. 7,060,808
U.S. Patent Publication No. 20160296616

The present invention particularly relates to the following items:
1. A composition comprising an amorphous, substantially solid film having an average thickness of 0.005 to 5mm, which is soluble in an aqueous solution, said film comprising a biologically active polypeptide, a sugar and an amphipathic surfactant.
2. The composition of item 1, wherein the sugar is selected from group consisting of: glucose, dextrose, fructose, lactose, maltose, xylose, sucrose, corn sugar syrup, sorbitol, hexitol, maltilol, xylitol, mannitol, melezitose, raffinose, and a combination thereof.
3. The composition of item 1, wherein the amphipathic surfactant comprises poly(maleic anhydride-alt-1 octadecene).
4. The composition of item 1, wherein the poly(maleic anhydride-alt-1 octadecene) is substituted with 3-(dimethylamino) propylamine.
5. The composition of item 1, further comprising a buffer.
6. The composition of item 1, further comprising a water-soluble polymer.
7. The composition of item 1, further comprising a salt.
8. The composition of item 1, wherein the biologically active polypeptide is substantially non-immunogenic when administered to a human.
9. The composition of item 1, wherein the biologically active polypeptide is a human polypeptide or a humanized antibody.
10. The composition of item 1, wherein the biologically active polypeptide is PEGylated.
11. The composition of item 1, wherein the biologically active polypeptide is an antibody.
12. The composition of item 1, wherein the antibody is a monoclonal antibody.
13. The composition of item 12, wherein the monoclonal antibody is a humanized antibody.
14. The composition of item 1, wherein the biologically active polypeptide is an enzyme, a ligand or a receptor.
15. The composition of item 14, wherein the biologically active polypeptide is an enzyme.
16. The composition of item 14, wherein the biologically active polypeptide is a cytokine.
17. The composition of item 14, wherein biologically active polypeptide is the extra cellular domain of a receptor.
18. The composition of item 1, further comprising HPMC.
19. The composition of item 1, further comprising tragacanthum gum.
20. The composition of item 1, further comprising sorbitol.
21. The composition of item 1, further comprising HPMC, tragacanthum gum and sorbitol.
22. The composition of item 1, further comprising a pH buffering agent.
23. The composition of item 22, wherein the pH buffering agent comprises a Tris, citrate or phosphate buffer.
24. The composition of item 1, further comprising sucrose.
25. The composition of item 1, wherein the composition is produced by a method comprising:
   (i) providing an aqueous solution comprising the biologically active polypeptide, the sugar and the amphipathic surfactant; and
   (ii) drying the solution sufficiently to form an amorphous, substantially solid film, which is soluble in an aqueous solution.
26. The composition of item 25, wherein the drying step is performed at an ambient temperature.
27. A composition comprising an amorphous substantially solid film comprising a biologically active polypeptide, a sugar and an amphipathic surfactant, said composition produced by a method comprising:
   (i) providing an aqueous solution comprising the biologically active polypeptide, the sugar and the amphipathic surfactant; and
   (ii) drying the solution sufficiently to form an amorphous, substantially solid film, which is soluble in an aqueous solution.
28. The composition of item 27, wherein the drying step is performed at an ambient temperature.
29. The composition of item 27, wherein the drying step is performed at 0.1 to 3.0 atm of pressure.
30. A method of providing biologically active polypeptide to a subject comprising administering an effective amount of a composition according to item 1 to the subject.
31. The method of item 30, further comprising reconstituting the composition in an aqueous solution prior to administration.
32. The method of item 31, wherein the aqueous solution is administered via injection.
33. The method of item 31, wherein the aqueous solution is sterile.
34. The method of item 31, wherein the aqueous solution is a pH buffered solution.
35. The method of item 31, comprising administering the composition orally.

## Claims

1. A composition comprising an amorphous, substantially solid film comprising a biologically active polypeptide, a sugar, and an amphipathic surfactant, wherein the biologically active polypeptide is selected from factors acting on the immune system, an anti-infective agent, an antibody, an enzyme, a ligand, and a receptor, and wherein the amphipathic surfactant comprises poly(maleic anhydride-alt-1 octadecene) substituted with 3-(dimethylamino) propylamine.

2. The composition of claim 1, wherein the biologically active polypeptide is substantially non-immunogenic when administered to a human.

3. The composition of claim 1 or 2, wherein the biologically active polypeptide makes up from 0.1 to 30% of the total solids of the composition.

4. The composition of any one of claims 1 to 3, wherein the composition comprises 0.1% to 10% of the poly(maleic anhydride-alt-1 octadecene) substituted with 3-(dimethylamino) propylamine.

5. The composition of any one of claims 1 to 4, wherein the sugar is glucose, dextrose, fructose, lactose, maltose, xylose, sucrose, corn sugar syrup, sorbitol, hexitol, maltitol, xylitol, mannitol, melezitose, raffinose, or a combination thereof; preferably, wherein the sugar is sorbitol, and wherein the composition comprises 0.1% to 7% of said sorbitol.

6. The composition of any one of claims 1 to 5, further comprising:
(i) a buffer, a water-soluble polymer, a salt, or a combination thereof; or
(ii) HPMC, tragacanthum gum, sorbitol, or a combination thereof; or
(iii) a pH buffering agent, sucrose, or a combination thereof.

7. The composition of any one of claims 1 to 6, wherein the film has an average thickness of 0.005 to 5 mm, and/or the film is soluble in an aqueous solution.

8. The composition of any one of claims 1 to 7, wherein the composition is producible by a method comprising:
(i) providing an aqueous solution comprising the biologically active polypeptide, the sugar and the amphipathic surfactant; and
(ii) drying the solution sufficiently to form an amorphous, substantially solid film.

9. The composition of any one of claims 1 to 7 producible by the method defined in claim 8, wherein the drying step is performed at an ambient temperature and/or at 0.1 to 3.0 atm of pressure.

10. A composition comprising an amorphous substantially solid film comprising a biologically active polypeptide, a sugar, and an amphipathic surfactant, wherein the biologically active polypeptide is selected from factors acting on the immune system, an anti-infective agent, an antibody, an enzyme, a ligand, and a receptor, and wherein the amphipathic surfactant comprises poly(maleic anhydride-alt-1 octadecene) substituted with 3-(dimethylamino) propylamine, said composition being producible by a method comprising:
(i) providing an aqueous solution comprising the biologically active polypeptide, the sugar and the amphipathic surfactant; and
(ii) drying the solution sufficiently to form an amorphous, substantially solid film.

11. The composition of claim 10, wherein the composition comprises 0.1% to 10% of the poly(maleic anhydride-alt-1 octadecene) substituted with 3-(dimethylamino) propylamine.

12. The composition of claim 10 or 11, wherein the sugar is glucose, dextrose, fructose, lactose, maltose, xylose, sucrose, corn sugar syrup, sorbitol, hexitol, maltitol, xylitol, mannitol, melezitose, raffinose, or a combination thereof; preferably, wherein the sugar is sorbitol, and wherein the composition comprises 0.1% to 7% of said sorbitol.

13. The composition according to any one of claims 1 to 12 for use in providing biologically active polypeptide to a subject, said use comprising administering an effective amount of said composition to the subject.

14. A method of storing a biologically active polypeptide comprising:
(i) providing an aqueous solution comprising the biologically active polypeptide, a sugar, and an amphipathic surfactant, wherein the biologically active polypeptide is selected from factors acting on the immune system, an anti-infective agent, an antibody, an enzyme, a ligand, and a receptor, and wherein the amphipathic surfactant comprises poly(maleic anhydride-alt-1 octadecene) substituted with 3-(dimethylamino) propylamine;
(ii) drying the solution sufficiently to form an amorphous, substantially solid film comprising the biologically active polypeptide; and
(iii) storing the substantially solid film.

15. The method of claim 14, wherein:
(i) the composition comprises 0.1% to 10% of the poly(maleic anhydride-alt-1 octadecene) substituted with 3-(dimethylamino) propylamine; and/or
(ii) the sugar is glucose, dextrose, fructose, lactose, maltose, xylose, sucrose, corn sugar syrup, sorbitol, hexitol, maltitol, xylitol, mannitol, melezitose, raffinose, or a combination thereof; preferably, wherein the sugar is sorbitol, and wherein the composition comprises 0.1% to 7% of said sorbitol.
